# EUROPÄISCHE PATENTSCHRIFT

(11) **EP 1 972 290 B1**
(45) Veröffentlichungstag und Bekanntmachung des Hinweises auf die Patenterteilung: **28.09.2016**
(21) Anmeldenummer: 08152874.7
(22) Anmeldetag: 18.03.2008
(51) Int. Cl.: A61B 17/86, A61B 50/00, A61B 50/30, A61B 50/20

(54) **Haltevorrichtung für ein Implantat**
Retaining device for an implant
Dispositif de maintien pour un implant

(30) Priorität: 22.03.2007 DE 102007015154
(43) Veröffentlichungstag der Anmeldung: 24.09.2008
(73) Patentinhaber: Aesculap AG, 78532 Tuttlingen (DE)
(72) Erfinder: Löffler, Burkhard, 79868 -Feldberg (Falkau) (DE); Beger, Jens, 78532 Tuttlingen (DE); Celmerowski, Beate, 78549 Spaichingen (DE); Fischer, Kay, 78532 Tuttlingen (DE)
(74) Vertreter: Hoeger, Stellrecht & Partner Patentanwälte mbB

(56) Entgegenhaltungen:
- WO-A-02/30315
- WO-A-99/52465
- WO-A-2009/024189
- FR-A- 2 785 033

## Beschreibung

Die vorliegende Erfindung betrifft eine Haltevorrichtung für ein Implantat, mit einer ersten Verbindungseinrichtung zum lösbaren Verbinden der Haltevorrichtung mit dem Implantat, wobei die erste Verbindungseinrichtung aus einer ersten Verbindungsstellung, in der ein Implantat mit der Haltevorrichtung verbindbar oder verbunden ist, in eine erste Lösestellung, in der die Haltevorrichtung das Implantat freigibt, überführbar ist.

Die vorliegende Erfindung betrifft auch eine Lagereinheit zum Aufnehmen und/oder Festlegen mindestens einer Haltevorrichtung für ein Implantat, welche Lagereinheit eine Haltevorrichtung umfasst, wobei die Haltevorrichtung eine erste Verbindungseinrichtung zum lösbaren Verbinden der Haltevorrichtung mit dem Implantat umfasst, wobei die erste Verbindungseinrichtung aus einer ersten Verbindungsstellung, in der ein Implantat mit der Haltevorrichtung verbindbar oder verbunden ist, in eine erste Lösestellung, in der die Haltevorrichtung das Implantat freigibt, überführbar ist.

Eine Haltevorrichtung für ein Implantat, mit einer ersten Verbindungseinrichtung zum lösbaren Verbinden der Haltevorrichtung mit dem Implantat ist beispielsweise aus der US 6,929,646 B2 bekannt. Diese Haltevorrichtung weist einen länglichen Griffabschnitt auf, mit dessen Hilfe ein mit dem Griffabschnitt verbundenes Implantat in eine gewünschte Implantierposition und -lage relativ zu einem zu operierenden Körperteil gebracht werden kann. Nach Befestigung des Implantats an dem Körperteil kann der Griffabschnitt von dem Implantat abgetrennt werden.

Ferner sind aus der FR 2 785 033 A1 (Basis für den Oberbegriff des Anspruchs 1), der WO 99/52465 A1 sowie der WO 02/30315 A1 Haltevorrichtungen sowie Lagereinheiten der eingangs beschriebenen Arten bekannt.

Es hat sich herausgestellt, dass die Organisation und Handhabung insbesondere sehr kleiner Implantate problematisch ist. Diese Implantate unterscheiden sich zum Teil nur sehr geringfügig voneinander, müssen jedoch für eine bestimmte Operation in zahlreichen Varianten bereitgestellt werden, so dass ein Chirurg im Verlauf einer Operation entscheiden kann, welches oder welche der insgesamt zur Verfügung stehenden Implantate verwendet werden sollen. In einigen Ländern ist es außerdem erforderlich, genau zu dokumentieren, welche und wie viele Implantate im Verlauf einer Operation verwendet wurden.

Hiervon ausgehend ist es Aufgabe der Erfindung, eine Haltevorrichtung und eine Lagereinheit der eingangs beschriebenen Art so zu verbessern, dass sich zweifelsfrei feststellen lässt, ob ein Implantat mit der Haltevorrichtung verbunden war und von der Haltevorrichtung gelöst wurde.

Diese Aufgabe wird bei einer Haltevorrichtung der eingangs beschriebenen Art erfindungsgemäß dadurch gelöst, dass die Haltevorrichtung eine Anzeigevorrichtung aufweist, die eine zumindest einmalige Überführung der ersten Verbindungseinrichtung aus der ersten Verbindungsstellung in die erste Lösestellung anzeigt.

Eine solche Anzeigevorrichtung ermöglicht es, zweifelsfrei festzustellen, dass ein Implantat mit der Haltevorrichtung verbunden war und von der Haltevorrichtung gelöst wurde. Hieraus kann gefolgert werden, dass dieses Implantat im Verlauf einer Operation verwendet wurde. Die Zuordnung dieses Implantats kann mithilfe jener Haltevorrichtung erfolgen, deren Anzeigevorrichtung die Überführung der ersten Verbindungseinrichtung aus der ersten Verbindungsstellung in die erste Lösestellung anzeigt. Es versteht sich, dass die beschriebene Anzeigevorrichtung auch bei Haltevorrichtungen vorgesehen sein kann, die nur eine erste Verbindungseinrichtung zum lösbaren Verbinden der Haltevorrichtung mit einem Implantat aufweisen und keine, wie nachfolgend beschrieben, zweite Verbindungseinrichtung zum lösbaren Verbinden der Haltevorrichtung mit einer Lagereinheit.

Günstig ist es, insbesondere auch bei einer Haltevorrichtung der eingangs beschriebenen Art, wenn die Haltevorrichtung eine zweite Verbindungseinrichtung zum lösbaren Verbinden der Haltevorrichtung mit einer Lagereinheit aufweist. Die Haltevorrichtung ermöglicht es also mithilfe einer ersten Verbindungseinrichtung, ein Implantat oder mehrere Implantate an der Haltevorrichtung lösbar festzulegen. Mithilfe der zweiten Verbindungseinrichtung der Haltevorrichtung kann diese lösbar mit einer Lagereinheit verbunden werden. Eine solche Haltevorrichtung erlaubt es, für jedes Implantat oder für eine Gruppe von Implantaten eine eigene Haltevorrichtung bereitzustellen, so dass jedes Implantat oder jede Gruppe von Implantaten mithilfe der zugeordneten Haltevorrichtung einfacher gehandhabt werden kann. Die Haltevorrichtungen können mit der Lagereinheit verbunden werden, so dass sich die Implantate übersichtlich anordnen lassen und mithilfe der Lagereinheit gemeinsam gehandhabt werden können.

Vorzugsweise sind die erste Verbindungseinrichtung und die zweite Verbindungseinrichtung voneinander unabhängig betätigbar. Somit kann ein Implantat mithilfe der ersten Verbindungseinrichtung mit der Haltevorrichtung verbunden werden und von der Haltevorrichtung gelöst werden, indem die erste Verbindungseinrichtung betätigt wird. Diese Betätigung ist unabhängig davon, ob die Haltevorrichtung mithilfe der zweiten Verbindungseinrichtung mit der Lagereinheit verbunden oder von dieser gelöst ist. In entsprechender Weise kann die zweite Verbindungseinrichtung betätigt werden, um die Haltevorrichtung mit der Lagereinheit zu verbinden oder die Haltevorrichtung von der Lagereinheit zu lösen, ohne dass dies einen Einfluss auf die erste Verbindungseinrichtung und somit auf die Verbindung zwischen einem Implantat und der Haltevorrichtung hat.

Vorzugsweise ist die erste Verbindungseinrichtung aus einer ersten Verbindungsstellung, in der ein Implantat mit der Haltevorrichtung verbindbar oder verbunden ist, in eine erste Lösestellung, in der die Haltevorrichtung das Implantat freigibt, überführbar. Somit kann das Implantat in der ersten Verbindungsstellung der ersten Verbindungseinrichtung zuverlässig an der Haltevorrichtung fixiert und in der ersten Lösestellung der ersten Verbindungseinrichtung in einfacher Weise von der Haltevorrichtung entfernt werden.

Vorzugsweise ist die erste Verbindungseinrichtung derart ausgebildet, dass zur Überführung der ersten Verbindungseinrichtung aus der ersten Verbindungsstellung in die erste Lösestellung eine erste Lösekraft erforderlich ist. Durch die erste Lösekraft ist der Widerstand definiert, der für eine Trennung des Implantats von der Haltevorrichtung überwunden werden muss. Es empfiehlt sich, eine Lösekraft vorzusehen, die so hoch ist, dass sich ein Implantat nicht unabsichtlich von der Haltevorrichtung lösen kann, zum Beispiel wenn die Haltevorrichtung bei einem Transport leichten Erschütterungen ausgesetzt ist. Andererseits soll die erste Lösekraft klein genug sein, um ein Implantat manuell oder mithilfe eines Entnahmewerkzeugs von der Haltevorrichtung lösen zu können.

Vorzugsweise umfasst die Haltevorrichtung eine erste Rückstelleinrichtung, die die erste Verbindungseinrichtung aus der ersten Lösestellung in die erste Verbindungsstellung überführt. Mithilfe der ersten Rückstelleinrichtung kann eine Vorzugsstellung der ersten Verbindungseinrichtung definiert werden, die der ersten Verbindungsstellung entspricht. Diese Vorzugsstellung kann unabhängig davon eingenommen werden, ob ein Implantat an der Haltevorrichtung gehalten ist oder nicht.

Günstig ist es, wenn die zweite Verbindungseinrichtung aus einer zweiten Verbindungsstellung, in der die Haltevorrichtung mit der Lagereinheit verbindbar oder verbunden ist, in eine zweite Lösestellung, in der die Haltevorrichtung von der Lagereinheit lösbar ist, überführbar ist. Auf diese Weise kann die Haltevorrichtung in der zweiten Verbindungsstellung der zweiten Verbindungseinrichtung zuverlässig an der Lagereinheit festgelegt werden, so dass die Haltevorrichtung nicht selbst gehandhabt werden muss, sondern mithilfe der Lagereinheit gehandhabt werden kann. In der zweiten Lösestellung der zweiten Verbindungseinrichtung kann die Haltevorrichtung von der Lagereinheit gelöst werden, so dass die Haltevorrichtung unabhängig von der Lagereinheit gehandhabt werden kann.

Vorzugsweise ist die zweite Verbindungseinrichtung derart ausgebildet, dass zur Überführung der zweiten Verbindungseinrichtung aus der zweiten Verbindungsstellung in die zweite Lösestellung eine zweite Lösekraft erforderlich ist. Diese zweite Lösekraft sollte so hoch sein, dass ein unbeabsichtigtes Lösen der Haltevorrichtung von der Lagereinheit vermieden werden kann. Die zweite Lösekraft sollte andererseits so klein sein, dass sich die Haltevorrichtung vorzugsweise ohne Zuhilfenahme von Werkzeugen von der Lagereinheit entfernen lässt.

Weiterhin ist es bevorzugt, wenn die Haltevorrichtung eine zweite Rückstelleinrichtung umfasst, die die zweite Verbindungseinrichtung aus der zweiten Lösestellung in die zweite Verbindungsstellung überführt. Mithilfe der zweiten Rückstelleinrichtung kann eine Vorzugsstellung der zweiten Verbindungseinrichtung definiert werden, die der zweiten Verbindungsstellung entspricht. Dabei kann eine Überführung in diese Vorzugsstellung unabhängig davon erfolgen, ob die Haltevorrichtung mit der Lagereinheit verbunden ist oder nicht.

Besonders bevorzugt ist es, dass sich die erste Lösekraft und die zweite Lösekraft nach Betrag und/oder Richtung voneinander unterscheiden. Hierdurch kann eine unbeabsichtigte gleichzeitige Betätigung der ersten Verbindungseinrichtung und der zweiten Verbindungseinrichtung vermieden werden. Somit ist gewährleistet, dass bei Aufbringen der ersten Lösekraft nur die erste Verbindungseinrichtung aus der ersten Verbindungsstellung in die erste Lösestellung gebracht wird, ohne dass dies einen Einfluss auf den Zustand der zweiten Verbindungseinrichtung hat. In entsprechender Weise bewirkt ein Aufbringen der zweiten Lösekraft eine Überführung der zweiten Verbindungseinrichtung aus der zweiten Verbindungsstellung in die zweite Lösestellung, ohne dass dies den Zustand der ersten Verbindungseinrichtung beeinflusst.

Besonders bevorzugt ist es, wenn die erste Lösekraft und die zweite Lösekraft voneinander linear unabhängig sind. Dies ermöglicht es, durch Wahl der entsprechenden Löserichtung zu entscheiden, ob die erste Verbindungseinrichtung aus der ersten Verbindungsstellung in die erste Lösestellung oder ob die zweite Verbindungseinrichtung aus der zweiten Verbindungsstellung in die zweite Lösestellung gebracht werden soll. Durch die linear unabhängigen ersten und zweiten Löserichtungen kann ausgeschlossen werden, dass eine der beiden Verbindungseinrichtungen in unbeabsichtigter Weise aus ihrer Verbindungsstellung in ihre Lösestellung gebracht wird. Dies gilt unabhängig davon, ob die erste Lösekraft kleiner, gleich oder größer ist als die zweite Lösekraft.

Bevorzugt ist ferner, dass die erste Lösekraft kleiner ist als die zweite Lösekraft. Dies ermöglicht es, die Lösekräfte so einzustellen, dass selbst dann, wenn die Lösekräfte gleich gerichtet sein sollten, zunächst die erste Verbindungseinrichtung aus der ersten Verbindungsstellung in die erste Lösestellung gebracht wird, und dann erst die zweite Verbindungseinrichtung aus der zweiten Verbindungsstellung in die zweite Lösestellung gebracht wird.

Vorzugsweise ist die erste Verbindungseinrichtung derart ausgebildet, dass das Implantat bei einer Bewegung aus einer ersten Halteposition, in der das Implantat mit der Haltevorrichtung verbunden ist, in eine erste Freigabeposition, in der das Implantat von der Haltevorrichtung gelöst ist, in einer ersten Handhabungsrichtung handhabbar ist. Mit der Handhabungsrichtung kann vorgegeben werden, in welcher Richtung ein Chirurg das Implantat handhaben muss, um es von der Haltevorrichtung zu trennen.

Ferner ist die zweite Verbindungseinrichtung vorzugsweise derart ausgebildet, dass die Haltevorrichtung bei einer Bewegung aus einer zweiten Halteposition, in der die Haltevorrichtung mit der Lagereinheit verbunden ist, in eine zweite Freigabeposition, in der die Haltevorrichtung von der Lagereinheit gelöst ist, in einer zweiten Handhabungsrichtung handhabbar ist. Mithilfe der zweiten Handhabungsrichtung kann definiert werden, in welcher Richtung die Haltevorrichtung gehandhabt werden muss, um sie von der Lagereinheit zu trennen.

Besonders bevorzugt ist es, wenn die erste Handhabungsrichtung und die zweite Handhabungsrichtung voneinander linear unabhängig sind. Wenn ein Chirurg ein Implantat entsprechend der ersten Handhabungsrichtung von der Haltevorrichtung löst, ist durch die lineare Unabhängigkeit der Handhabungsrichtungen ausgeschlossen, dass gleichzeitig die Haltevorrichtung von der Lagereinheit gelöst wird. In entsprechender Weise ist gewährleistet, dass bei Wahl der zweiten Handhabungsrichtung zum Lösen der Haltevorrichtung von der Lagereinheit ein gegebenenfalls mit der Haltevorrichtung verbundenes Implantat nicht von der Haltevorrichtung gelöst wird.

Besonders bevorzugt ist es, wenn die erste Handhabungsrichtung und die zweite Handhabungsrichtung zueinander senkrecht oder im Wesentlichen senkrecht sind. Dies ermöglicht eine besonders einfache Handhabung des Implantats, der Haltevorrichtung und der Lagereinheit, bei der ein unbeabsichtigtes Lösen des Implantats von der Haltevorrichtung und ein unbeabsichtigtes Lösen der Haltevorrichtung von der Lagereinheit ausgeschlossen ist.

Vorzugsweise definiert die Haltevorrichtung eine Halteachse, die die Position und/oder die Lage des Implantats vorgibt, wenn dieses mit der Haltevorrichtung verbunden ist. Auf diese Weise ist es möglich, eine absolute Raumposition und/oder Raumlage eines Implantats zu definieren, wenn die Haltevorrichtung mit einer Lagereinheit verbunden ist.

Besonders bevorzugt ist es, wenn die Halteachse und die erste Handhabungsrichtung zueinander senkrecht oder im Wesentlichen senkrecht sind. Dies ermöglicht eine besonders einfache und schonende Überführung des Implantats aus der ersten Halteposition in die erste Freigabeposition.

Günstig ist es, wenn die Halteachse und die zweite Handhabungsrichtung zueinander parallel oder im Wesentlichen parallel sind. Dies ermöglicht eine platzsparende Anordnung des Implantats an der Haltevorrichtung und der Haltevorrichtung an der Lagereinheit.

In bevorzugter Weise umfasst die erste Verbindungseinrichtung mindestens ein Halteelement, das ausgebildet ist, in der ersten Verbindungsstellung der ersten Verbindungseinrichtung das Implantat mit der Haltevorrichtung zu verbinden. Ein solches Halteelement kann eine nur einmalige Überführung der ersten Verbindungseinrichtung aus der ersten Verbindungsstellung in die erste Lösestellung ermöglichen. Ein solches Halteelement kann auch einen mehrmaligen Wechsel zwischen der ersten Verbindungsstellung und der ersten Lösestellung ermöglichen.

Eine bevorzugte Ausführungsform der Erfindung sieht vor, dass das mindestens eine Halteelement zungenförmig ist. Dies ermöglicht eine federnde Bewegung des Halteelements zwischen der ersten Verbindungsstellung und der ersten Lösestellung.

Zusätzlich oder optional kann das mindestens eine Halteelement auch kreissegmentförmig sein. Hierdurch lassen sich Implantate besonders zuverlässig mit der Haltevorrichtung verbinden, die Implantatabschnitte aufweisen, die der Kreissegmentform eines Halteelements entsprechend geformt sind. Ein kreissegmentförmiges Halteelement kann außerdem gegebenenfalls ein Lösen des Implantats von der Haltevorrichtung in einer von der ersten Handhabungsrichtung abweichenden Richtung verhindern.

Bevorzugt ist es, wenn das mindestens eine Halteelement innerhalb einer Halteebene beweglich und/oder verformbar ist. Durch die Beweglichkeit und/oder Verformbarkeit des mindestens einen Halteelements innerhalb der Halteebene kann der Betrag der ersten Lösekraft, die für die Überführung der ersten Verbindungseinrichtung aus der ersten Verbindungsstellung in die erste Lösestellung erforderlich ist, besonders genau definiert werden.

Vorzugsweise ist die Halteebene zu der Halteachse senkrecht oder im Wesentlichen senkrecht. Dies ermöglicht es insbesondere bei einem im Wesentlichen länglichen Implantat, beispielsweise einer Knochenschraube, dieses in einer ersten Handhabungsrichtung von der Haltevorrichtung zu lösen, die zu der Halteachse senkrecht ist. Hierdurch wird erreicht, dass das Implantat und die Haltevorrichtung bei einer Überführung des Implantats aus der ersten Halteposition in die erste Freigabeposition nur einem minimalen Reibkontakt ausgesetzt sind.

Günstig ist es, wenn die erste Verbindungseinrichtung mindestens zwei Halteelemente aufweist. Dies ermöglicht es, die für eine Überführung der ersten Verbindungseinrichtung aus der ersten Verbindungsstellung in die erste Lösestellung erforderliche erste Lösekraft in mindestens zwei Halteelemente, die während der genannten Überführung bewegt und/oder verformt werden, einzuleiten. Auf diese Weise kann die mechanische Belastung der einzelnen Halteelemente minimiert werden.

Vorzugsweise sind die mindestens zwei Halteelemente zum Überführen der ersten Verbindungseinrichtung aus der ersten Verbindungsstellung in die erste Lösestellung in zueinander entgegengesetzten Öffnungsrichtungen bewegbar. Auf diese Weise kann die erste Lösekraft gleichmäßig auf die mindestens zwei Halteelemente verteilt werden.

Weiterhin ist es bevorzugt, wenn die mindestens zwei Halteelemente zum Überführen der ersten Verbindungseinrichtung aus der ersten Lösestellung in die erste Verbindungsstellung in zueinander entgegengesetzten Schließrichtungen bewegbar sind. Dies ermöglicht eine schonende und selbstzentrierende Überführung des Implantats aus der ersten Freigabeposition in die erste Halteposition.

Besonders bevorzugt ist es, wenn mindestens ein Halteelement zur Ausbildung der ersten Rückstelleinrichtung bei Überführung der ersten Verbindungseinrichtung aus der ersten Verbindungsstellung in die erste Lösestellung eine erste Rückstellkraft aufbaut, mit der die erste Verbindungseinrichtung zurück in die erste Verbindungsstellung überführbar ist. Dies kann beispielsweise durch Wahl eines entsprechenden Materials, beispielsweise Kunststoff, gewährleistet werden, so dass das Halteelement elastisch verformbar ist und bei Auslenkung aus einer Grundstellung, die der ersten Verbindungsstellung entspricht, eine erste Rückstellkraft aufbauen kann. Hierdurch ist ein besonders einfacher Aufbau der ersten Rückstelleinrichtung gewährleistet.

Günstig ist es, wenn das mindestens eine Halteelement eine Implantataufnahme zur Aufnahme des Implantats begrenzt. Somit trägt das Halteelement zu einer exakten Positionierung eines Implantats an der Haltevorrichtung bei.

Günstig ist es ferner, wenn die Implantataufnahme einen Hinterschnitt aufweist. Dies ermöglicht eine besonders zuverlässige Verbindung des Implantats mit der Haltevorrichtung.

Eine Ausführungsform der Erfindung sieht vor, dass die Implantataufnahme umfangsseitig geschlossen ist. Dies ermöglicht eine besonders zuverlässige Fixierung des Implantats an der Haltevorrichtung.

Eine Ausführungsform der Erfindung sieht vor, dass die erste Verbindungseinrichtung mindestens ein Anlageelement umfasst, das in der ersten Verbindungsstellung der ersten Verbindungseinrichtung unter Vorspannung an das Implantat anlegbar ist. Mit einem solchen Anlageelement lässt sich auf besonders einfache Art und Weise erreichen, dass ein Implantat spielfrei an der Haltevorrichtung festgelegt werden kann, ohne dass die erste Verbindungseinrichtung der Haltevorrichtung hohen Toleranzanforderungen genügen muss.

Nach einer Ausführungsform der Erfindung begrenzt das Anlagesegment die Implantataufnahme. Hierdurch kann eine kompakte Haltevorrichtung geschaffen werden, die eine spielfreie Verbindung der Haltevorrichtung mit einem Implantat ermöglicht.

Eine Ausführungsform der Erfindung sieht vor, dass das mindestens eine Anlageelement kreissegmentförmig ist. Ein solches Anlageelement lässt sich besonders gut unter Herstellung eines großflächigen Kontakts an einen gekrümmten Implantatabschnitt anlegen.

Vorzugsweise umfasst die Anzeigevorrichtung mindestens ein Anzeigeelement, das bei einer Überführung der ersten Verbindungseinrichtung aus der ersten Verbindungsstellung in die erste Lösestellung zerstörbar und/oder plastisch verformbar ist. Dies ermöglicht eine besonders einfache Ausgestaltung der Anzeigevorrichtung.

Bevorzugt ist es, wenn die Anzeigevorrichtung mindestens einen Verbindungsabschnitt zur Verbindung von mindestens zwei Anzeigeelementen und/oder zur Verbindung des mindestens einen Anzeigeelements mit einem weiteren Teil der Haltevorrichtung aufweist, wobei der Verbindungsabschnitt bei einer Überführung der ersten Verbindungseinrichtung aus der ersten Verbindungsstellung in die erste Lösestellung durchtrennbar ist. Dies ermöglicht einen besonders einfachen Aufbau der Anzeigevorrichtung. Der Verbindungsabschnitt kann insbesondere eine Sollbruchstelle umfassen oder durch eine Sollbruchstelle gebildet sein.

Günstig ist es, wenn das mindestens eine Anzeigeelement bändchenförmig ist. Dies ermöglicht eine Zerstörung und/oder plastische Verformung des Anzeigeelements mithilfe relativ kleiner Zerstörungs- und/oder Verformungskräfte.

Eine Ausführungsform der Erfindung sieht vor, dass das mindestens eine Anzeigeelement relativ zueinander bewegliche Elementabschnitte ausweist, die sich in zueinander winklig stehenden Ebenen erstrecken. Auf diese Weise kann die für ein Auslösen der Anzeigevorrichtung erforderliche Auslösekraft besonders gut eingestellt werden.

Nach einer Ausführungsform der Erfindung ist das mindestens eine Anzeigeelement durch ein Halteelement gebildet. Dies ermöglicht einen besonders einfachen Aufbau der Haltevorrichtung. Darüber hinaus ist in besonders zuverlässiger Weise gewährleistet, dass bei Aufbringen der ersten Lösekraft zur Überführung der ersten Verbindungseinrichtung aus der ersten Verbindungsstellung in die erste Lösestellung auch die Anzeigevorrichtung ausgelöst wird.

Günstig ist es, wenn die Haltevorrichtung einen plattenförmigen Grundkörper umfasst. Der Grundkörper ermöglicht eine besonders kompakte Ausgestaltung der Haltevorrichtung.

Bevorzugt ist es, wenn sich der Grundkörper in der Halteebene erstreckt. Dies ermöglicht eine Raum sparende Anordnung der Halteelemente an dem Grundkörper.

Weiterhin ist es bevorzugt, wenn sich der Grundkörper senkrecht oder im Wesentlichen senkrecht zu der Halteachse erstreckt. Dies ermöglicht eine Raum sparende Anordnung eines Implantats an der Haltevorrichtung sowie eine Raum sparende Anordnung der Haltevorrichtung an der Lagereinheit.

Günstig ist es, wenn das mindestens eine Halteelement und/oder das mindestens eine Anlageelement und/oder das mindestens eine Anzeigeelement an dem Grundkörper angeordnet ist oder sind. Hierdurch kann eine besonders kompakte Haltevorrichtung geschaffen werden.

Vorzugsweise ist oder sind das mindestens eine Halteelement und/oder das mindestens eine Anlageelement und/oder das mindestens eine Anzeigeelement mit dem Grundkörper einstückig ausgebildet. Dies ermöglicht eine besonders kostengünstige Herstellung der Haltevorrichtung.

Vorzugsweise umfasst die Haltevorrichtung einen Datenspeicher zur Speicherung von Implantat-Daten. Dies ermöglicht eine eindeutige Zuordnung eines mit der Haltevorrichtung verbindbaren Implantats und der Haltevorrichtung. Die Implantat-Daten können sich beispielsweise auf einen Hersteller, eine Artikelnummer, eine Chargennummer und/oder auf sonstige Eigenschaften des Implantats beziehen. Durch Auslesen des Datenspeichers ist es möglich, die Implantat-Daten auch dann nachvollziehen zu können, wenn das Implantat bereits von der Haltevorrichtung gelöst wurde. Dies erleichtert es, nachzuvollziehen, welches Implantat im Verlauf einer Operation verwendet wurde.

Günstig ist es, wenn der Datenspeicher unlösbar mit der Haltevorrichtung verbunden ist. Dies erleichtert die Zuordnung der Implantat-Daten zu einem mit der Haltevorrichtung verbundenen oder von der Haltevorrichtung gelösten Implantat.

Eine Ausführungsform der Erfindung sieht vor, dass der Datenspeicher mehrfach beschreibbar ist. Dies ermöglicht es, die Haltevorrichtung für unterschiedliche Implantate oder für eine Gruppe von unterschiedlichen Implantaten zu verwenden.

Besonders bevorzugt ist es, wenn der Datenspeicher zur Anzeige der Implantat-Daten in Form eines optischen Datenspeichers ausgebildet ist. Dies ermöglicht eine optische Identifizierung insbesondere von sehr kleinen Implantaten, die gegebenenfalls keine für eine Anzeige von Implantat-Daten genügend große Flächen aufweisen.

Günstig ist es, wenn die Implantat-Daten in alphanumerischer Form, als Barcode und/oder als Matrixcode vorliegen. Somit können die Implantat-Daten ohne Zuhilfenahme weiterer Vorrichtungen direkt abgelesen und/oder beispielsweise mithilfe eines Scanners bequem erfasst werden.

Besonders bevorzugt ist es, wenn der Datenspeicher zur Anzeige der Implantat-Daten eine Sichtfläche umfasst. Dies ermöglicht ein einfaches Ablesen oder Scannen der Implantat-Daten.

Besonders bevorzugt ist es, wenn die Sichtfläche an dem Grundkörper ausgebildet ist, so dass sich der Aufbau der Haltevorrichtung weiter vereinfacht.

Günstig ist es, wenn die Implantat-Daten mit der Haltevorrichtung einstückig ausgebildet sind. Hierdurch muss ein Datenspeicher nicht separat bereitgestellt werden. Wenn die Haltevorrichtung beispielsweise in einem Spritzgussverfahren hergestellt wird, können die Implantat-Daten durch entsprechende Gestaltung der Spritzgussform im selben Herstellungsvorgang mit hergestellt werden.

Eine Ausführungsform der Erfindung sieht vor, dass der Datenspeicher in Form eines elektronischen Datenspeichers ausgebildet ist. Ein solcher Datenspeicher ermöglicht es, auch sehr umfangreiche Implantat-Daten speichern zu können.

Eine Ausführungsform der Erfindung sieht vor, dass der Datenspeicher mindestens ein RFID-Element umfasst. Ein solches Element lässt sich preisgünstig in die Haltevorrichtung integrieren oder an dieser anordnen, beispielsweise durch Einspritzen in ein Kunststoffmaterial. Zudem kann ein RFID-Element mithilfe eines geeigneten Lesegeräts berührungslos ausgelesen werden, um die Implantat-Daten auszulesen.

Günstig ist es, wenn die zweite Verbindungseinrichtung mindestens ein Verbindungselement umfasst, das ausgebildet ist, in der zweiten Verbindungsstellung der zweiten Verbindungseinrichtung die Haltevorrichtung mit der Lagereinheit zu verbinden. Mithilfe des mindestens einen Verbindungselements kann die Haltevorrichtung zuverlässig mit der Lagereinheit verbunden und einfach von der Lagereinheit gelöst werden.

Besonders bevorzugt ist es, wenn das mindestens eine Verbindungselement zur Ausbildung einer Rastverbindung mindestens ein Rastelement umfasst oder als Rastelement ausgebildet ist, wobei das Rastelement in der zweiten Verbindungsstellung der zweiten Verbindungseinrichtung mit der Lagereinheit rastend in Eingriff steht oder in Eingriff bringbar ist. Ein solches Rastelement ermöglicht eine besonders zuverlässige Verbindung der Haltevorrichtung mit der Lagereinheit. Darüber hinaus kann die Überführung der zweiten Verbindungseinrichtung aus der zweiten Lösestellung in die zweite Verbindungsstellung mit einem entsprechenden Rastvorgang des Rastelements einhergehen, wodurch einer Bedienperson eine gute akustische und/oder optische Rückmeldung darüber vermittelt wird, dass die zweite Verbindungseinrichtung ihre zweite Verbindungsstellung erreicht hat.

Vorzugsweise ist das mindestens eine Verbindungselement innerhalb einer Verbindungsebene beweglich und/oder verformbar. Auf diese Weise lässt sich eine für eine Überführung der zweiten Verbindungseinrichtung aus der zweiten Verbindungsstellung in die zweite Lösestellung gegebenenfalls erforderliche zweite Lösekraft hinsichtlich Betrag und/oder Richtung genau definieren.

Vorzugsweise ist die Verbindungsebene zu der Halteachse des Implantats parallel oder im Wesentlichen parallel. Dies ermöglicht einen besonders einfachen Aufbau der zweiten Verbindungseinrichtung.

Weiterhin ist es vorteilhaft, wenn die zweite Verbindungseinrichtung mindestens zwei Verbindungselemente aufweist. Hierdurch kann die für eine Überführung der zweiten Verbindungseinrichtung aus der zweiten Verbindungsstellung in die zweite Lösestellung gegebenenfalls erforderliche zweite Lösekraft in mehrere Verbindungselemente eingeleitet werden, so dass die mechanische Belastung der einzelnen Verbindungselemente minimiert wird.

Vorzugsweise sind die mindestens zwei Verbindungselemente zum Überführen der zweiten Verbindungseinrichtung aus der zweiten Verbindungsstellung in die zweite Lösestellung in zueinander entgegengesetzten Öffnungsrichtungen bewegbar. Dies ermöglicht eine komfortable Handhabung der Haltevorrichtung bei der Überführung der zweiten Verbindungseinrichtung aus der zweiten Verbindungsstellung in die zweite Lösestellung.

Weiter vorzugsweise sind die mindestens zwei Verbindungselemente zum Überführen der zweiten Verbindungseinrichtung aus der zweiten Lösestellung in die zweite Verbindungsstellung in zueinander entgegengesetzten Schließrichtungen bewegbar. Dies ermöglicht eine komfortable Handhabung der Haltevorrichtung bei der Überführung der zweiten Verbindungseinrichtung aus der zweiten Lösestellung in die zweite Verbindungsstellung.

Eine bevorzugte Ausführungsform der Erfindung sieht vor, dass das mindestens eine Verbindungselement zur Ausbildung der zweiten Rückstelleinrichtung bei Überführung der zweiten Verbindungseinrichtung aus der zweiten Verbindungsstellung in die zweite Lösestellung eine zweite Rückstellkraft aufbaut, mit der die zweite Verbindungseinrichtung zurück in die zweite Verbindungsstellung überführbar ist. Dies ermöglicht einen besonders einfachen Aufbau der zweiten Rückstelleinrichtung, die bewirkt, dass die zweite Verbindungsstellung der zweiten Verbindungseinrichtung die Vorzugsstellung der zweiten Verbindungseinrichtung ist.

Vorzugsweise begrenzt das mindestens eine Verbindungselement in der zweiten Verbindungsstellung der zweiten Verbindungseinrichtung einen Hinterschnittbereich zur Aufnahme eines Abschnitts der Lagereinheit. Hierdurch ist eine besonders zuverlässige Verbindung zwischen der Haltevorrichtung und der Lagereinheit gewährleistet.

Bevorzugt ist es, wenn der Hinterschnittbereich durch eine Anlagefläche des Grundkörpers begrenzt ist. Auf diese Weise kann mithilfe des Grundkörpers eine weitere, der zweiten Verbindungseinrichtung zugeordnete Funktion realisiert werden.

Eine Ausführungsform der Erfindung sieht vor, dass die zweite Verbindungseinrichtung mindestens einen im Wesentlichen U-förmigen Materialabschnitt umfasst, der zwei sich parallel oder im Wesentlichen parallel zu der Halteachse erstreckende U-Schenkel aufweist, die über eine U-Basis miteinander verbunden sind. Ein solcher Materialabschnitt ermöglicht eine einfache Anordnung und/oder Realisierung mindestens eines Verbindungselements.

Nach einer Ausführungsform der Erfindung ist die Halteachse zwischen zwei U-Schenkeln angeordnet. Auf diese Weise kann das Implantat mithilfe der U-Schenkel vor mechanischen Einflüssen geschützt werden, wenn das Implantat mit der Haltevorrichtung verbunden ist.

Eine weitere Ausführungsform sieht vor, dass die Halteachse außerhalb eines zwischen den U-Schenkeln ausgebildeten Raums angeordnet ist. Dies ermöglicht einen besonders kompakten Aufbau der zweiten Verbindungseinrichtung.

Vorzugsweise umfasst die zweite Verbindungseinrichtung mindestens ein Betätigungselement zum Überführen der zweiten Verbindungseinrichtung aus der zweiten Verbindungsstellung in die zweite Lösestellung. Hierdurch kann die Haltevorrichtung in besonders einfacher Art und Weise von der Lagereinheit gelöst werden.

Vorzugsweise ist das mindestens eine Betätigungselement in Form eines Griffabschnitts ausgebildet. Hierdurch kann die zweite Verbindungseinrichtung manuell betätigt werden.

In vorteilhafter Weise ist das mindestens eine Betätigungselement an einem freien Ende eines U-Schenkels angeordnet. Dies ermöglicht eine besonders einfache Überführung der zweiten Verbindungseinrichtung aus der zweiten Verbindungsstellung in die zweite Lösestellung.

Weiter vorzugsweise ist das mindestens eine Betätigungselement an dem Grundkörper angeordnet. Hierdurch wird die Handhabung der Haltevorrichtung erleichtert.

Besonders bevorzugt ist es, wenn entlang der Halteachse des Implantats gesehen mindestens ein Betätigungselement und mindestens ein Verbindungselement der zweiten Verbindungseinrichtung auf einander gegenüberliegenden Seiten des Grundkörpers angeordnet sind. Hierdurch kann die zweite Verbindungseinrichtung relativ zu der Lagereinheit in einem vergleichsweise schwer zugänglichen, aber gut geschützten Raum der Lagereinheit angeordnet werden, während das Betätigungselement auf einer entgegengesetzten Seite des Grundkörper gut zugänglich angeordnet werden kann.

Vorzugsweise umfasst die Haltevorrichtung mindestens eine Sicherungseinrichtung, die ein Implantieren des Implantats verhindert, wenn das Implantat mit der Haltevorrichtung verbunden ist. Dies hat den Vorteil, dass ein Implantat, das mit der Haltevorrichtung verbunden ist, im Verlauf einer Operation nicht unabsichtlich zusammen mit der Haltevorrichtung an einem zu operierenden Körperteil verwendet werden kann.

Besonders günstig ist es, wenn die Sicherungseinrichtung mindestens einen zu der Halteachse beabstandeten Sicherungsabschnitt umfasst. Dies ermöglicht es, einen Funktionsbereich des Implantats, beispielsweise einen Gewindeabschnitt, mithilfe des Sicherungsabschnitts abzuschirmen, so dass dieser Funktionsbereich nicht in Eingriff mit einem zu operierenden Körperteil gebracht werden kann.

Vorzugsweise ist der Sicherungsabschnitt zu dem Grundkörper winklig, insbesondere senkrecht oder im Wesentlichen senkrecht angeordnet. Dies ermöglicht einen besonders kompakten Aufbau der Haltevorrichtung.

Günstig ist es ferner, wenn der Sicherungsabschnitt in einem Querschnitt senkrecht zu der Halteachse im Wesentlichen C-förmig ist. Dies erlaubt es, einen Funktionsbereich eines Implantats besonders großflächig abschirmen zu können. Darüber hinaus erlaubt die Sicherungseinrichtung einen weitgehenden mechanischen Schutz des Implantats.

Vorzugsweise ist der Sicherungsabschnitt in Querschnitten senkrecht zu der Halteachse in einem zu dem Grundkörper benachbarten Bereich größer als in einem von dem Grundkörper entfernten Bereich. Dies ermöglicht eine besonders einfache Positionierung der Haltevorrichtung an der Lagereinheit. Hierbei kann die Haltevorrichtung zunächst mit seinem von dem Grundkörper entfernten Bereich an die Lagereinheit herangeführt und dann mit dem zu dem Grundkörper benachbarten Bereich in Anlage mit der Lagereinheit gebracht werden.

Nach einer Ausführungsform der Erfindung ist der Sicherungsabschnitt durch den U-förmigen Materialabschnitt der zweiten Verbindungseinrichtung gebildet. Hierdurch ist es möglich, auf einen separaten Sicherungsabschnitt verzichten zu können. Mithilfe der U-Basis dieses Materialabschnitts kann ein mit der Haltevorrichtung verbundenes Implantat auch in einer zu der Halteachse parallelen Richtung vor mechanischen Einflüssen geschützt werden.

Günstig ist es, wenn die Haltevorrichtung eine Führungseinrichtung aufweist, mit der die Haltevorrichtung relativ zu der Lagereinheit positionierbar ist. Dies erleichtert die Handhabung der Haltevorrichtung bei der Verbindung der Haltevorrichtung mit der Lagereinheit.

Günstig ist es, wenn die Führungseinrichtung mindestens einen Führungsabschnitt aufweist, der zur Anlage an einen Abschnitt der Lagereinheit ausgebildet ist. Der Führungsabschnitt kann durch bereits beschriebene Teile der Haltevorrichtung gebildet sein, beispielsweise durch Teile der zweiten Verbindungseinrichtung und/oder durch einen Sicherungsabschnitt der Sicherungseinrichtung. Die Führungseinrichtung kann jedoch zusätzlich oder optional auch mindestens einen separaten Führungsabschnitt aufweisen.

Wenn die Haltevorrichtung mindestens Implantat umfasst, kann eine Baugruppe bereitgestellt werden, die mit einer Lagereinheit verbunden werden kann. Diese Baugruppe kann für eine Operation bereitgestellt und bei Nichtgebrauch wieder sterilisiert und für die nächste Operation bereitgestellt werden.

Die eingangs gestellte Aufgabe wird ferner bei einer Lagereinheit der eingangs beschriebenen Art erfindungsgemäß dadurch gelöst, dass die Haltevorrichtung eine Anzeigevorrichtung aufweist, die eine zumindest einmalige Überführung der ersten Verbindungseinrichtung aus der ersten Verbindungsstellung in die erste Lösestellung anzeigt.

Eine solche Anzeigevorrichtung ermöglicht es, zweifelsfrei festzustellen, dass ein Implantat mit der Haltevorrichtung verbunden war und von der Haltevorrichtung gelöst wurde. Hieraus kann gefolgert werden, dass dieses Implantat im Verlauf einer Operation verwendet wurde. Die Zuordnung dieses Implantats kann mithilfe jener Haltevorrichtung erfolgen, deren Anzeigevorrichtung die Überführung der ersten Verbindungseinrichtung aus der ersten Verbindungsstellung in die erste Lösestellung anzeigt. Es versteht sich, dass die beschriebene Anzeigevorrichtung auch bei Haltevorrichtungen vorgesehen sein kann, die nur eine erste Verbindungseinrichtung zum lösbaren Verbinden der Haltevorrichtung mit einem Implantat aufweisen und keine, wie nachfolgend beschrieben, zweite Verbindungseinrichtung zum lösbaren Verbinden der Haltevorrichtung mit einer Lagereinheit.

Vorteilhaft ist es, wenn die Haltevorrichtung eine zweite Verbindungseinrichtung zum lösbaren Verbinden der Haltevorrichtung mit der Lagereinheit aufweist. Die Lagereinheit erleichtert die Handhabung mindestens einer Haltevorrichtung und somit auch die Handhabung eines gegebenenfalls mit der Haltevorrichtung verbundenen, insbesondere sehr kleinen Implantats. Besonders vorteilhaft ist es, wenn die Lagereinheit eine Aufnahme und/oder ein Festlegen mehrerer Haltevorrichtungen erlaubt.

Bevorzugt ist es, wenn die Lagereinheit für eine zueinander identische Orientierung von mindestens zwei Haltevorrichtungen ausgebildet ist. Auf diese Weise wird die Auffindbarkeit einer bestimmten Haltevorrichtung und somit eines bestimmten Implantats erleichtert.

Ferner ist es bevorzugt, wenn die Lagereinheit für eine regelmäßige Anordnung von mindestens drei Haltevorrichtungen ausgebildet ist. Auch dies erleichtert die Auffindbarkeit bestimmter Haltevorrichtungen und bestimmter Implantate.

Eine besonders übersichtliche Anordnung der Haltevorrichtung wird erreicht, wenn die Lagereinheit für eine Anordnung der Haltevorrichtungen in Reihen- oder Spalten ausgebildet ist. Dies ermöglicht außerdem eine Raum sparende Anordnung mehrerer Haltevorrichtungen an der Lagereinheit.

Günstig ist es, wenn die Lagereinheit zum Aufnehmen und/oder Festlegen mindestens einer Haltevorrichtung mindestens eine Aufnahme umfasst. Mithilfe der Aufnahme kann die Relativposition und/oder -lage der Haltevorrichtung relativ zu der Lagereinheit definiert werden.

Besonders bevorzugt ist es, wenn die mindestens eine Haltevorrichtung zumindest abschnittsweise in die mindestens eine Aufnahme einsetzbar ist. Hierdurch können die Haltevorrichtungen Raum sparend an der Lagereinheit angeordnet und zuverlässig mit dieser verbunden werden.

Vorzugsweise umfasst die Lagereinheit zur Ausbildung oder Anordnung der mindestens einen Aufnahme eine Platte. Dies ermöglicht eine preisgünstige Herstellung der Lagereinheit, die sich außerdem besonders gut reinigen lässt.

Vorzugsweise ist die mindestens eine Aufnahme durch einen Abschnitt der Platte begrenzt, der mit der zweiten Verbindungseinrichtung der Haltevorrichtung verbindbar ist. Hierdurch kann eine besonders einfach aufgebaute Lagereinheit geschaffen werden.

Günstig ist es, wenn die Aufnahme einen Querschnitt aufweist, der die Drehlage einer Haltevorrichtung um deren Halteachse relativ zu der Lagereinheit vorgibt. Auf diese Weise kann die Drehlage der Haltevorrichtung vorgegeben werden, so dass sich die Auffindbarkeit dieser Haltevorrichtung und somit eines bestimmten Implantats verbessert.

Besonders günstig ist es, wenn die mindestens eine Aufnahme einen Querschnitt in Form eines Langlochs aufweist. Hierdurch kann eine Vorzugsorientierung der Haltevorrichtung relativ zu der Lagereinheit in einfacher Weise vorgegeben werden.

Eine Ausführungsform der Erfindung sieht vor, dass die Aufnahme mindestens ein von der Platte hervorstehendes Lagerelement zur Verbindung der Lagereinheit mit der zweiten Verbindungseinrichtung einer Haltevorrichtung aufweist. Mithilfe eines solchen Lagerelements lässt sich eine besonders zuverlässige Verbindung zwischen der Haltevorrichtung und der Lagereinheit herstellen.

Günstig ist es, wenn die Lagereinheit mindestens eine Abstandseinrichtung aufweist, die die Platte zu einer Aufstellfläche für die Lagereinheit beabstandet. Dies ermöglicht es, einen Abstand zwischen der Platte und der Aufstellfläche zu schaffen, in der sich zumindest Abschnitte der Haltevorrichtungen und/oder der Implantate anordnen lassen, ohne dass diese mit der Aufstellfläche in Berührung kommen.

Bevorzugt ist es, dass die Abstandseinrichtung eine sich entlang der Berandung der Platte zumindest abschnittsweise erstreckende Berahmung umfasst. Hierdurch kann die Lagereinheit besonders kippsicher auf der Aufstellfläche aufgestellt werden. Wenn die Berahmung sich entlang der gesamten Berandung der Platte erstreckt, kann ein sich zwischen der Platte der Lagereinheit und der Aufstellfläche erstreckender Raum, in dem Haltevorrichtungen zumindest abschnittsweise angeordnet sein können, besonders gut vor mechanischen Einflüssen geschützt werden.

Eine Baugruppe aus einer Lagereinheit mit mindestens einer Haltevorrichtung ermöglicht es, für eine bestimmte Operation benötigte Haltevorrichtungen und/oder Implantate übersichtlich und ohne weitere Vorbereitungsschritte bereitstellen zu können. Diese Baugruppe kann nach einer Operation wieder vervollständigt, sterilisiert und für eine Folgeoperation bereitgestellt werden.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht einer Lagereinheit und einer Vielzahl von Haltevorrichtungen, die mit der Lagereinheit verbunden oder von dieser gelöst sind, sowie einer Vielzahl von Implantaten, die mit jeweils einer Haltevorrichtung verbunden sind;
- Figur 2:: eine perspektivische Ansicht einer nicht erfindungsgemäßen Haltevorrichtung gemäß einer ersten Ausführungsform;
- Figur 3:: eine Explosionsansicht der Haltevorrichtung gemäß Figur 2, eines Implantats sowie eines Ausschnitts der Lagereinheit gemäß Figur 1;
- Figur 4:: eine perspektivische Ansicht der in Figur 3 dargestellten Teile, wobei das Implantat lösbar mit der Haltevorrichtung verbunden ist, wobei die Haltevorrichtung lösbar mit der Lagereinheit verbunden ist;
- Figur 5:: eine der Figur 4 entsprechende Ansicht, wobei das Implantat mithilfe eines Entnahmewerkzeugs von der Haltevorrichtung gelöst wird;
- Figur 6:: eine perspektivische Ansicht einer nicht erfindungsgemäßen Haltevorrichtung gemäß einer zweiten Ausführungsform und eines Ausschnitts der Lagereinheit gemäß Figur 1;
- Figur 7:: eine Ansicht der Haltevorrichtung gemäß Figur 6 aus einer gegenüber Figur 6 um etwa 90° gedrehten Perspektive;
- Figur 8:: eine perspektivische Ansicht einer nicht erfindungsgemäßen Haltevorrichtung gemäß einer dritten Ausführungsform;
- Figur 9:: eine Ansicht der Haltevorrichtung gemäß Figur 8 aus einer gegenüber Figur 8 um etwa 90° gedrehten Perspektive;
- Figur 10:: eine perspektivische Ansicht einer nicht erfindungsgemäßen Haltevorrichtung gemäß einer vierten Ausführungsform;
- Figur 11:: eine Ansicht der Haltevorrichtung gemäß Figur 10 aus einer gegenüber Figur 10 um etwa 180° gedrehten Perspektive;
- Figur 12:: eine perspektivische Ansicht einer nicht erfindungsgemäßen Haltevorrichtung gemäß einer fünften Ausführungsform;
- Figur 13:: eine Ansicht der Haltevorrichtung gemäß Figur 12 aus einer gegenüber Figur 12 um etwa 120° gedrehten Perspektive;
- Figur 14:: eine perspektivische Ansicht einer erfindungsgemäßen Haltevorrichtung gemäß einer sechsten Ausführungsform;
- Figur 15:: eine Ansicht der Haltevorrichtung gemäß Figur 14 aus einer gegenüber Figur 14 um etwa 150° gedrehten Perspektive;
- Figur 16:: eine perspektivische Ansicht einer erfindungsgemäßen Haltevorrichtung gemäß einer siebten Ausführungsform;
- Figur 17:: eine Ansicht der Haltevorrichtung gemäß Figur 16 aus einer gegenüber Figur 16 um etwa 150° gedrehten Perspektive;
- Figur 18:: eine perspektivische Ansicht einer erfindungsgemäßen Haltevorrichtung gemäß einer achten Ausführungsform;
- Figur 19:: eine Ansicht der Haltevorrichtung gemäß Figur 18 aus einer gegenüber Figur 18 um etwa 90° gedrehten Perspektive;
- Figur 20:: eine perspektivische Ansicht einer nicht erfindungsgemäßen Haltevorrichtung gemäß einer neunten Ausführungsform; und
- Figur 21:: eine Ansicht der Haltevorrichtung gemäß Figur 20 aus einer gegenüber Figur 20 um etwa 150° gedrehten Perspektive.

Gleiche oder funktional äquivalente Elemente sind in allen Figuren mit denselben Bezugszeichen bezeichnet.

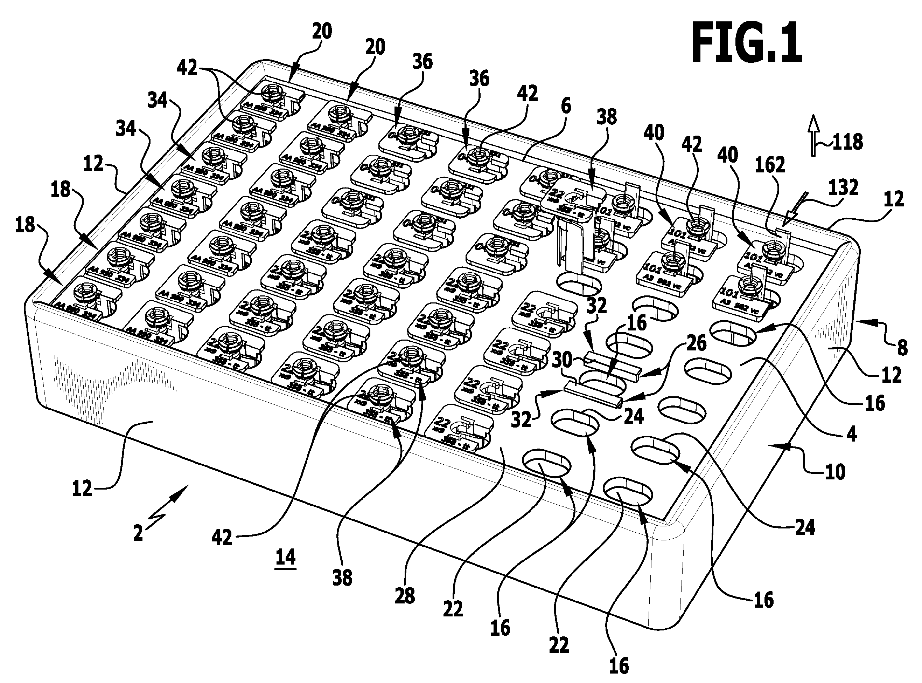

In Figur 1 ist eine zur Anordnung von Haltevorrichtungen für Implantate ausgebildete Lagereinheit insgesamt mit dem Bezugszeichen 2 bezeichnet. Diese weist eine rechteckförmige Platte 4 auf, deren Plattendicke wenige Millimeter beträgt. Entlang der Berandung 6 der Platte 4 erstreckt sich eine insgesamt mit dem Bezugszeichen 8 bezeichnete Abstandseinrichtung. Diese ist in Form einer Berahmung 10 ausgebildet, die vier jeweils paarweise zueinander senkrecht angeordnete und miteinander verbundene Wände 12 umfasst. Die Wände 12 erstrecken sich senkrecht zu der Platte 4 und beabstanden diese zu einer Aufstellfläche 14, auf der die Lagereinheit 2 aufgestellt ist.

Die Platte 4 weist eine Vielzahl von Aufnahmen 16 auf, die jeweils durch in der Platte 4 vorgesehene Aussparungen gebildet sind. Die Aufnahmen 16 sind regelmäßig über die Platte 4 verteilt und in zueinander parallelen Reihen 18 und hierzu senkrechten Spalten 20 angeordnet. Die Aufnahmen 16 sind jeweils in Form eines Langlochs ausgebildet, wobei die längere Querschnittsachse (ohne Bezugszeichen) in Richtung der Reihen 18 und die kürzere Querschnittsachse (ohne Bezugszeichen) in Richtung der Spalten 20 verläuft.

Die Platte 4 weist insgesamt 56 in sieben Reihen 18 und acht Spalten 20 angeordnete Aufnahmen 16 auf. Die Querschnitte 22 der Aufnahmen 16 sind gleich groß und zueinander identisch orientiert. Jede Aufnahme 16 ist durch Abschnitte 24 der Platte 4 begrenzt. Auf die Funktion der Abschnitte 24 wird weiter unten noch eingegangen.

Eine der in Figur 1 dargestellten Aufnahmen 16 umfasst zwei längliche Lagerelemente 26. Diese stehen von einer Oberseite 28 der Platte 4 hervor und erstrecken sich in Richtung der Reihen 18. Die Lagerelemente 26 umfassen zwei von der Oberseite 28 beabstandete und sich parallel zu der Platte 4 erstreckende Lagerabschnitte 30, die gemeinsam mit der Oberseite 28 der Platte 4 in Richtung der Reihen 18 verlaufende Lagerbereiche 32 begrenzen. Auch auf die Funktion der Lagerbereiche 32 wird weiter unten noch eingegangen.

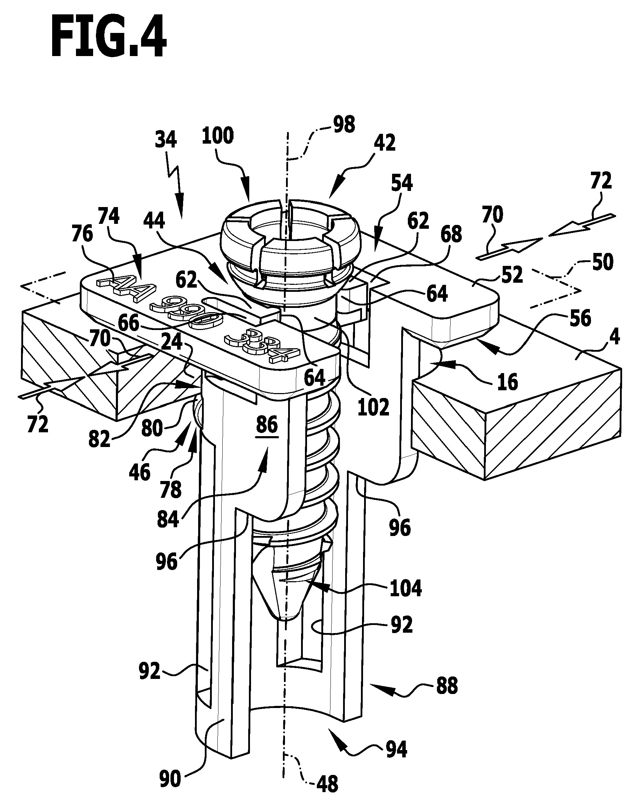

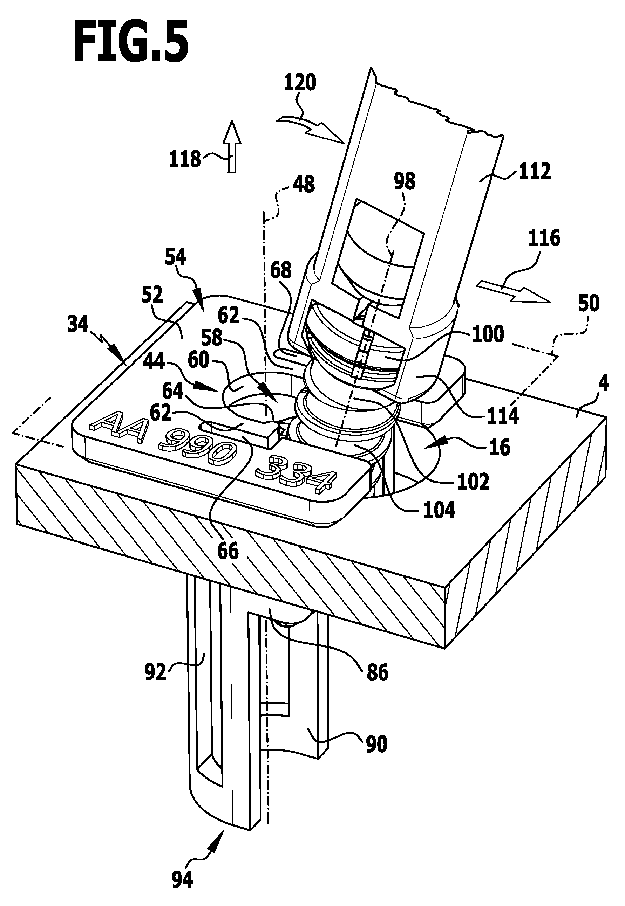

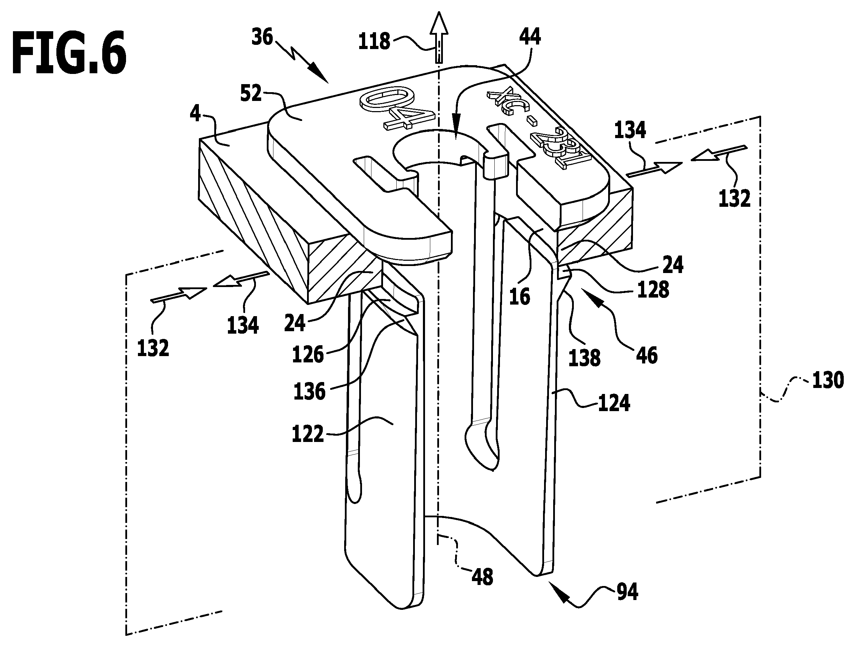

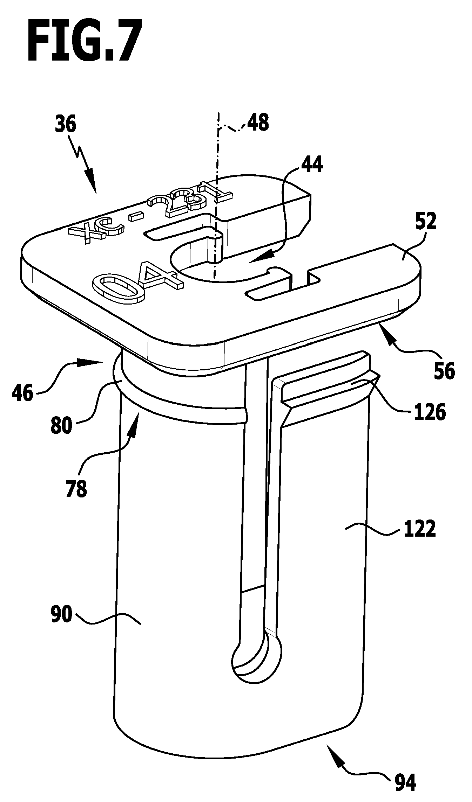

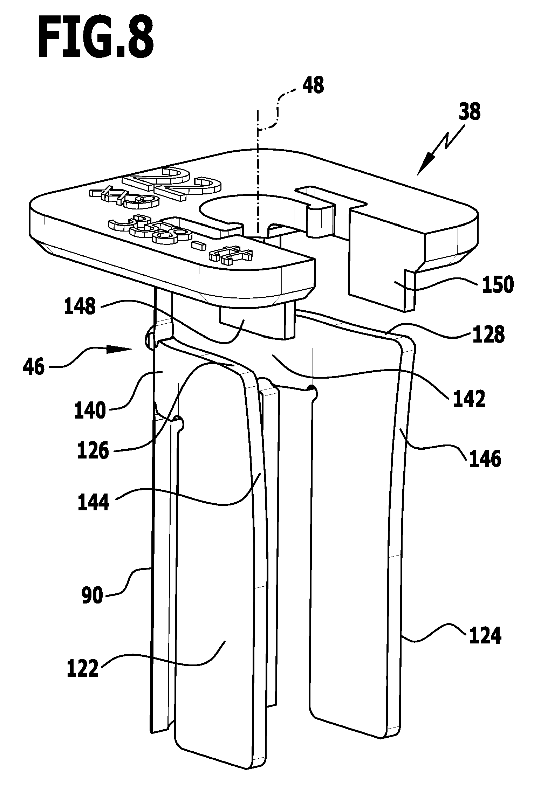

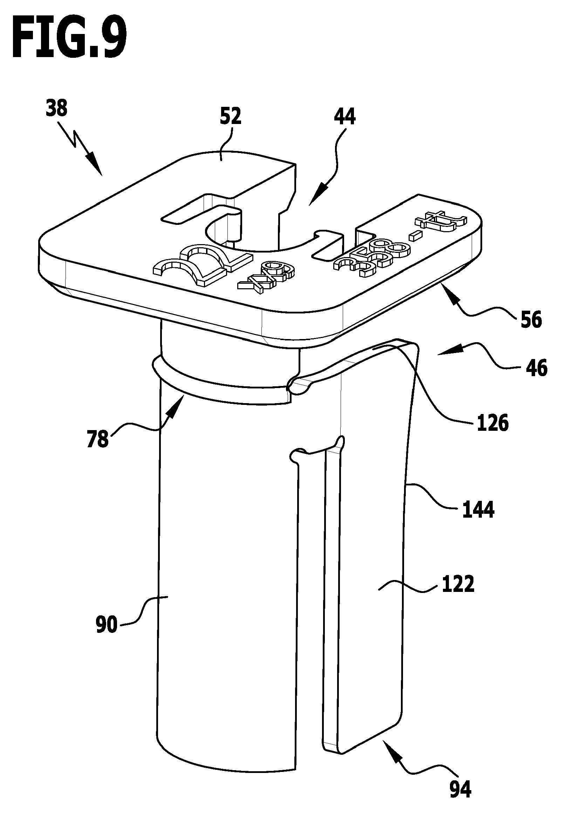

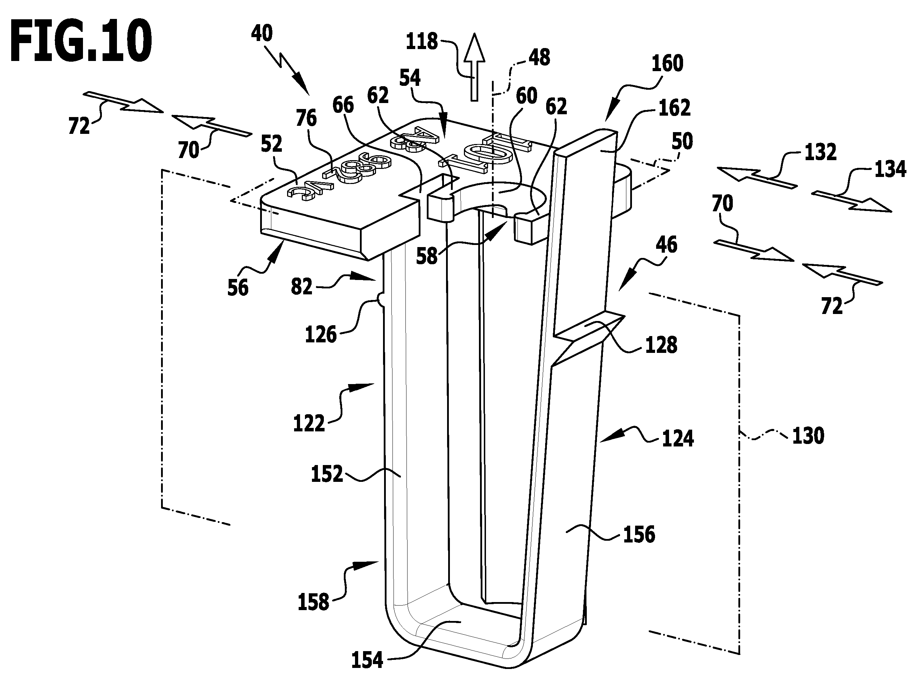

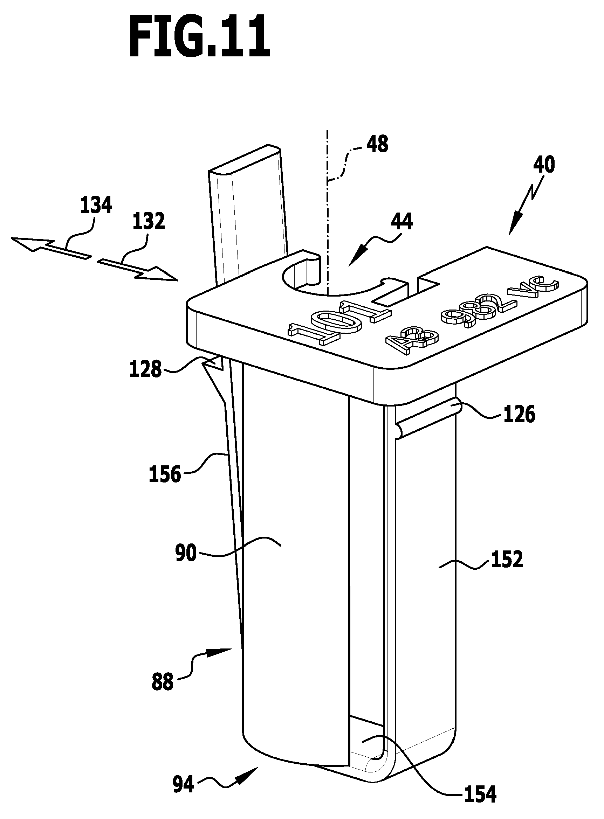

Die Lagereinheit 2 dient zum Aufnehmen und/oder Festlegen einer Vielzahl von Haltevorrichtungen, die mit den Aufnahmen 16 der Platte 4 lösbar verbunden werden können. In Figur 1 sind verschiedene Haltevorrichtungen dargestellt, die mit der Lagereinheit 2 lösbar verbunden sind, nämlich Haltevorrichtungen 34 (vergleiche Figuren 4 und 5), Haltevorrichtungen 36 (vergleiche Figuren 6 und 7), Haltevorrichtungen 38 (vergleiche Figuren 8 und 9) und Haltevorrichtungen 40 (vergleiche Figuren 10 und 11). In Figur 1 ist eine der Haltevorrichtungen 38 in einem von der Lagereinheit 2 gelösten Zustand dargestellt.

Jede der Haltevorrichtungen 34, 36, 38, 40 dient zur Anordnung eines in Form einer Schraube ausgebildeten Implantats 42. Jedes Implantat 42 kann, wie in Figur 1 dargestellt, lösbar mit einer der Haltevorrichtungen 34, 36, 38, 40 verbunden sein.

Die Haltevorrichtung 34 umfasst eine erste Verbindungseinrichtung 44 zum lösbaren Verbinden der Haltevorrichtung 34 mit einem Implantat 42 sowie eine zweite Verbindungseinrichtung 46 zum lösbaren Verbinden der Haltevorrichtung 34 mit der Lagereinheit 2.

Die Haltevorrichtung 34 definiert eine Halteachse 48, entlang der ein Implantat 42 angeordnet werden kann, wenn das Implantat 42 mit der Haltevorrichtung 34 verbunden ist und eine Halteposition einnimmt (siehe Figur 4). Diese Position des Implantats 42 wird im Folgenden als erste Halteposition bezeichnet.

Senkrecht zu der Halteachse 48 verläuft eine Halteebene 50, in der sich ein in etwa quadratischer, plattenförmiger Grundkörper 52 erstreckt. Dieser weist auf der der zweiten Verbindungseinrichtung 46 gegenüberliegenden Seite eine Sichtfläche 54 auf. Der Grundkörper 52 weist auf seiner der Sichtfläche 54 gegenüberliegenden Seite eine Anlagefläche 56 auf.

Die erste Verbindungseinrichtung 44 umfasst eine Implantataufnahme 58, die sich auf Höhe der Halteebene 50 im Bereich der Halteachse 48 erstreckt. Die Implantataufnahme 58 ist durch einen in etwa halbzylindrischen Anlageabschnitt 60 begrenzt, der von dem Grundkörper 52 gebildet ist. An den Anlageabschnitt 60 schließen sich zwei einander gegenüberliegend angeordnete, zungenförmige Halteelemente 62 an. Diese weisen an ihren freien Enden einander zugewandte Rastvorsprünge 64 auf. Die Rastvorsprünge 64 begrenzen die Implantataufnahme 58 derart, dass ein Hinterschnitt entsteht.

Der Grundkörper 52 weist auf den jeweils der Implantataufnahme 58 abgewandten Seiten der Halteelemente 62 längliche Freiräume 66 bzw. 68 auf. Die Freiräume 66 und 68 bewirken, dass sich die Halteelemente 62 in der Halteebene 50 federnd bewegen lassen. Die Halteelemente 62 können in einer Öffnungsrichtung 70 jeweils in die Freiräume 66 und 68 hinein bewegt werden. Die Halteelemente 62 können sich auch in einander zugewandten Schließrichtungen 72 jeweils in Richtung auf die Implantataufnahme 58 bewegen.

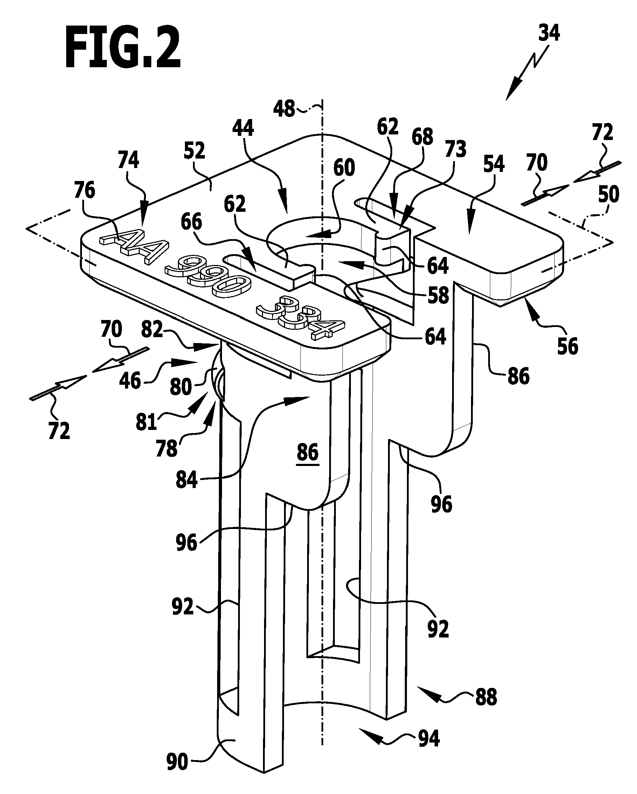

Die Halteelemente 72 sind in Figur 2 in einer ersten Verbindungsstellung der ersten Verbindungseinrichtung 44 dargestellt. Wenn die Halteelemente 72 durch Aufbringen einer ersten Lösekraft aus dieser ersten Verbindungsstellung heraus in die Freiräume 66 und 68 hinein ausgelenkt werden, kann die erste Verbindungseinrichtung 44 in eine erste Lösestellung überführt werden. Bei einer solchen Überführung der ersten Verbindungseinrichtung 44 aus der ersten Verbindungsstellung in die erste Lösestellung werden die Halteelemente 72 unter Auslenkung in die Freiräume 66 und 68 hinein elastisch verformt. Hierdurch bauen die Halteelemente 72 jeweils eine in Richtung auf die Implantataufnahme 58 gerichtete erste Rückstellkraft auf. Die ersten Rückstellkräfte bewirken, dass die Halteelemente nach Auslenkung in die Freiräume 66 und 68 von selbst wieder zurück in die in Figur 2 dargestellte Stellung gelangen. Auf diese Weise bilden die Halteelemente 72 eine erste Rückstelleinrichtung 73.

Der Grundkörper 52 umfasst einen insgesamt mit dem Bezugszeichen 74 bezeichneten Datenspeicher. Bei der Haltevorrichtung 34 umfasst der Datenspeicher 74 die Sichtfläche 54 des Grundkörpers 52. Auf der Sichtfläche 52 sind Implantat-Daten 76 angeordnet, die erhaben über die Sichtfläche 52 hervorstehen und zur Kennzeichnung eines Implantats 42 dienen, das über die erste Verbindungseinrichtung 44 mit der Haltevorrichtung 34 verbunden werden kann. Die Implantat-Daten 76 sind alphanumerisch.

Die zweite Verbindungseinrichtung 46, mit der die Haltevorrichtung 34 mit der Lagereinheit 2 verbunden werden kann, umfasst ein Verbindungselement 78 in Form eines gewölbten Rastelements 80. Das Rastelement 80 begrenzt gemeinsam mit der Anlagefläche 56 des Grundkörpers 52 einen Hinterschnittbereich 82, in dem ein auch in Figuren 1, 3 und 4 dargestellter Abschnitt 24 der Platte 4 der Lagereinheit 2 angeordnet werden kann.

Das Verbindungselement 78 ist in Figur 2 in einer zweiten Verbindungsstellung der zweiten Verbindungseinrichtung 46 dargestellt. Wenn das Verbindungselement 78 aus dieser zweiten Verbindungsstellung heraus durch Aufbringen einer zweiten Lösekraft in Richtung auf die Halteachse 48 verformt wird, kann die erste Verbindungseinrichtung 44 in eine zweite Lösestellung überführt werden. Bei einer solchen Überführung der zweiten Verbindungseinrichtung 46 aus der zweiten Verbindungsstellung in die zweite Lösestellung wird das Verbindungselement 78 elastisch verformt. Hierdurch baut das Verbindungselement 78 eine von der Halteachse 48 weg gerichtete zweite Rückstellkraft auf. Diese zweite Rückstellkraft bewirkt, dass das Verbindungselement 78 nach seiner Verformung in Richtung auf die Halteachse 78 von selbst wieder zurück in die in Figur 2 dargestellte Stellung gelangt. Auf diese Weise bildet das Verbindungselement 78 eine zweite Rückstelleinrichtung 81.

Die Haltevorrichtung 34 umfasst eine Führungseinrichtung 84, die zwei sich parallel zu der Halteachse 48 erstreckende, ebene Führungsabschnitte 86 aufweist. Die Führungsabschnitte 86 dienen zur Positionierung der Haltevorrichtung 34 relativ zu einer Aufnahme 16 der Lagereinheit 2.

Die Haltevorrichtung 34 umfasst ferner eine Sicherungseinrichtung 88, die einen Sicherungsabschnitt 90 umfasst, der in Querschnitten senkrecht zu der Halteachse 48 C-förmig ist. Der Sicherungsabschnitt 90 weist zwei in etwa rechteckige, parallel zu der Halteachse 48 verlaufende Schlitze 92 auf. Der Sicherungsabschnitt 90 weist ferner auf seiner dem Grundkörper 52 abgewandten Seite ein freies Ende 94 auf. Von diesem Ende aus in Richtung der Halteachse 48 gesehen vergrößern sich die senkrecht zu der Halteachse anliegenden Querschnitte des Sicherungsabschnitts 90 auf Höhe zweier Stufen 96. Hierdurch ist der Querschnitt der Sicherungseinrichtung 88 in einem zu dem Grundkörper 52 benachbarten Bereich größer als in einem von dem Grundkörper 52 entfernten Bereich.

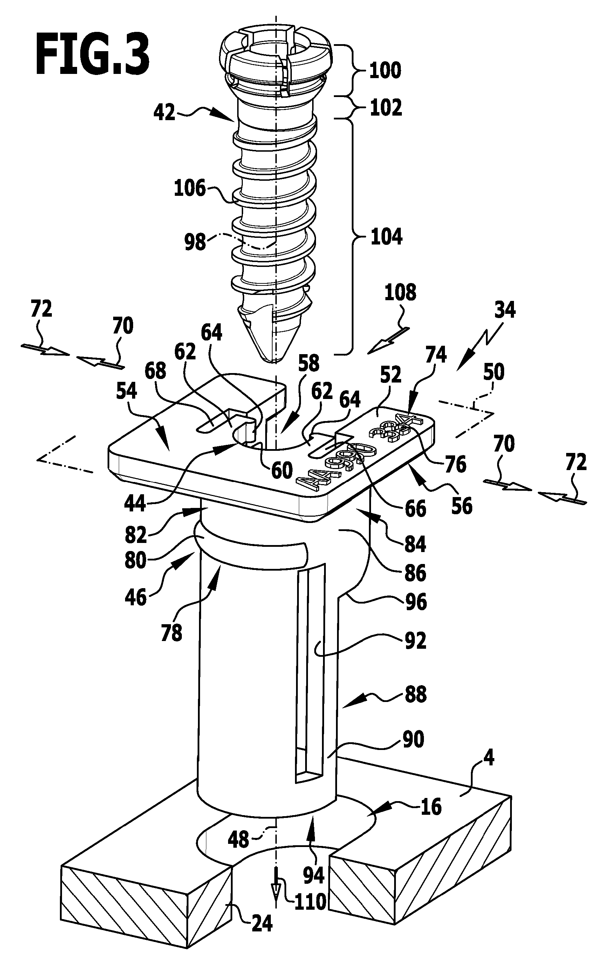

In Figur 3 ist die Haltevorrichtung 34 mit einem Implantat 42 dargestellt. Das Implantat 42 nimmt eine von der Haltevorrichtung 34 gelöste Position ein.

Das Implantat 42 erstreckt sich entlang einer Implantatachse 98. Es weist einen ersten Implantatabschnitt 100 in Form eines Schraubenkopfs auf. An diesen schließt sich ein kurzer zylindrischer Implantatabschnitt 102 an. Schließlich weist das Implantat 42 einen Implantatabschnitt 104 auf, der mit einem Außengewinde 106 versehen ist. Mithilfe des Außengewindes 106 kann das Implantat 42 an einem zu operierenden Körperteil befestigt werden.

Um das Implantat 42 mit der Haltevorrichtung 34 zu verbinden, kann der Implantatabschnitt 102 des Implantats 42 auf Höhe der Halteebene 50 seitlich zu der Haltevorrichtung 34 positioniert werden. Diese Position des Implantats 42 wird im Folgenden als erste Freigabeposition bezeichnet. Aus dieser Position heraus kann das Implantat 42 in mit 108 bezeichneter Verbindungsrichtung senkrecht zu der Halteachse 48 innerhalb der Halteebene 50 in die Implantataufnahme 58 hinein geschoben werden. Hierbei werden die Halteelemente 62 durch den Implantatabschnitt 102 in in zueinander entgegengesetzten Öffnungsrichtungen 70 auseinander bewegt, bis die Rastvorsprünge 64 den Implantatabschnitt 102 rastend umgreifen (vergleiche Figur 4).

Wenn das Implantat 42 seine in Figur 4 dargestellte erste Halteposition an der Haltevorrichtung 34 einnimmt, erstreckt sich der Implantatabschnitt 100 über die Sichtfläche 54 des Grundkörpers 52 hinaus. Der Implantatabschnitt 102 ist in der Implantataufnahme 58 gehalten. Der Implantatabschnitt 104 ist von dem in einem Querschnitt senkrecht zu der Halteachse 48 C-förmigen Sicherungsabschnitt 90 umgeben.

Die Haltevorrichtung 34 kann aus einer in Figur 3 dargestellten zweiten Freigabeposition in eine in Figur 4 dargestellte Position gebracht werden, in der die Haltevorrichtung 34 mit der Lagereinheit 2 verbunden ist. Diese Position der Haltevorrichtung 34 wird im Folgenden als zweite Halteposition bezeichnet.

Um die Haltevorrichtung 34 ausgehend von der zweiten Freigabeposition in die zweite Halteposition zu bringen, kann das freie Ende 94 des Sicherungsabschnitts 90 der Haltevorrichtung 34 in eine der Aufnahmen 16 der Platte 4 der Lagereinheit 2 eingeführt werden. Hierbei wird die Haltevorrichtung 34 in in Figur 3 mit Bezugszeichen 110 bezeichneter Verbindungsrichtung bewegt, bis das Rastelement 80 den Abschnitt 24 der Platte 4 rastend hintergreift und die Anlagefläche 56 des Grundkörper 52 an der Plattenoberseite 28 der Platte 4 anliegt (vergleiche Figur 4).

In Figur 4 nimmt die erste Verbindungseinrichtung 44 die erste Verbindungsstellung ein. Das Implantat 42 ist mit der Haltevorrichtung 34 verbunden und nimmt die erste Halteposition ein.

In Figur 4 nimmt die zweite Verbindungseinrichtung 46 die zweite Verbindungsstellung ein. Die Haltevorrichtung 34 ist mit der Lagereinheit 2 verbunden und nimmt die zweite Halteposition ein.

Die in Figur 4 dargestellte Anordnung ermöglicht es einem Chirurgen, das Implantat 42 einfach zu handhaben, da dieses mit der Haltevorrichtung 34 verbunden ist und diese wiederum mit der Lagereinheit 2 verbunden ist. Dabei kann das Implantat 42 mithilfe des Datenspeichers 74 eindeutig identifiziert werden.

Um das Implantat 42 aus seiner in Figur 4 dargestellten ersten Halteposition in die in Figur 5 dargestellte erste Freigabeposition zu bringen, kann ein Entnahmewerkzeug 112 verwendet werden. Das Entnahmewerkzeug 112 weist einen Werkzeugkopf 114 auf, der zum Umgreifen des Implantatabschnitts 104 ausgebildet ist. Das Entnahmewerkzeug 112 kann durch einen Schraubendreher gebildet sein, der auch für das Einschrauben des Außengewindes 106 des Implantats 42 in ein zu behandelndes Körperteil verwendet werden kann.

Um die erste Verbindungseinrichtung 44 aus ihrer in Figur 4 dargestellten ersten Verbindungsstellung in die erste Lösestellung zu bringen, kann das Entnahmewerkzeug 112 so bewegt werden, dass das Implantat 42 in einer in Figur 5 mit 116 bezeichneten ersten Handhabungsrichtung in zu der Halteachse 48 senkrechten Richtung gehandhabt wird, so dass sich der Implantatabschnitt 102 innerhalb der Halteebene 50 aus der Implantataufnahme 58 heraus bewegt. Hierbei muss eine erste Lösekraft aufgebracht werden, die durch den Widerstand der Halteelemente 62 bestimmt ist, die sich innerhalb der Halteebene 50 zur Freigabe des Implantatabschnitts 102 in Öffnungsrichtungen 70 (vergleiche Figur 3) in die angrenzenden Freiräume 66 und 68 hinein bewegen.

Sobald das Implantat 42 so weit aus der Implantataufnahme 58 heraus bewegt wurde, dass es die erste Freigabeposition einnimmt, kann das Implantat 42 in zu der Halteachse 48 paralleler Richtung von der Haltevorrichtung 34 entfernt werden. Hierbei verbleibt die Haltevorrichtung 34 an der Platte 4 der Lagereinheit 2. Dies liegt daran, dass die zweite Verbindungseinrichtung 46 von der ersten Verbindungseinrichtung 44 unabhängig betätigbar ist. Durch Aufbringen der ersten Lösekraft wird die erste Verbindungseinrichtung 44 aus der ersten Verbindungsstellung in die erste Lösestellung überführt. Hierbei verbleibt die zweite Verbindungseinrichtung 46 in der in den Figuren 4 und 5 dargestellten zweiten Verbindungsstellung, so dass die Haltevorrichtung 34 mit der Lagereinheit 2 verbunden bleibt.

Um nun die zweite Verbindungseinrichtung 46 in die zweite Lösestellung zu überführen, um die Haltevorrichtung 34 von der Lagereinheit 2 zu lösen, kann die Haltevorrichtung 34 in zu der Halteachse 48 paralleler Richtung in einer Handhabungsrichtung 118 gehandhabt und aus der Aufnahme 16 der Platte 4 heraus geschoben werden. Hierzu kann beispielsweise von dem freien Ende 94 des Sicherungsabschnitts 90 her in Richtung der zweiten Handhabungsrichtung 118 eine Druckkraft aufgebracht werden. Hierdurch kann eine zweite Lösekraft angebracht werden, die das Verbindungselement 78 unter Anlage an den Abschnitt 24 der Platte 4 in Richtung auf die Halteachse 48 verformt, so dass die zweite Verbindungseinrichtung 46 aus der zweiten Verbindungsstellung in die zweite Lösestellung überführt wird. Hierdurch kann die Haltevorrichtung 34 von der Platte 4 gelöst werden.

Die erste Handhabungsrichtung 116 und die zweite Handhabungsrichtung 118 verlaufen senkrecht zueinander. Es versteht sich, dass die Überführung der ersten Verbindungseinrichtung 44 aus der ersten Verbindungsstellung in die erste Lösestellung durch ein Verkippen des Implantats 42 um einen Kippwinkel 120 relativ zu der Halteachse 48 unterstützt werden kann. In diesem Fall können die erste Handhabungsrichtung 116 und die zweite Handhabungsrichtung 118 in einem Winkel zueinander orientiert sein, der einem rechten Winkel zuzüglich des Kippwinkels 120 entspricht.

Die in den Figuren 6 und 7 dargestellte Haltevorrichtung 36 weist einen zu der Haltevorrichtung 34 ähnlichen Aufbau auf. Im Folgenden wird lediglich auf die Unterschiede zwischen den Haltevorrichtungen 34 und 36 eingegangen. Im Unterschied zu der Haltevorrichtung 34 weist die Haltevorrichtung 36 nicht nur ein Verbindungselement 78 in Form eines gewölbten Rastelements 80 auf, sondern darüber hinaus zwei einander gegenüberliegend angeordnete Verbindungselemente 122 und 124. Diese erstrecken sich parallel zu der Halteachse 48 und sind an dem freien Ende 94 der Haltevorrichtung 36 mit dem Sicherungsabschnitt 90 verbunden.

Die Verbindungselemente 122 und 124 weisen an ihrem dem Grundkörper 52 der Haltevorrichtung 36 zugewandten Ende jeweils ein Rastelement 126 beziehungsweise 128 auf. Diese Rastelemente sind entlang der Halteachse 48 gesehen auf gleicher Höhe wie das Rastelement 80 angeordnet. Die Verbindungselemente 122 und 124 sind jeweils für sich und auch relativ zueinander innerhalb einer Verbindungsebene 130 beweglich und verformbar, so dass die Rastelemente 126 und 128 in zu einander entgegensetzten Öffnungsrichtungen 132 innerhalb der Verbindungsebene 130 aufeinander zu bewegt werden können. Hierdurch kann die zweite Verbindungseinrichtung 46 der Haltevorrichtung 36 aus ihrer in Figur 6 dargestellten zweiten Verbindungsstellung in die zweite Lösestellung überführt werden. Wenn die Verbindungselemente 122 und 124 im Bereich der Rastelemente 126 und 128 so nah zueinander beabstandet sind, dass die Rastelemente 126 und 128 mit den Abschnitten 24 der Platte 4 außer Eingriff gebracht werden können, kann die Haltevorrichtung 36 in zu der Halteachse 48 paralleler zweiter Handhabungsrichtung 118 aus der zweiten Halteposition in die zweite Freigabeposition (vergleiche Figur 7) gebracht werden.

Nachdem die Verbindungselemente 122 und 124 und die Rastelemente 126 und 128 außer Eingriff mit der Aufnahme 16 der Platte 4 gebracht wurden, bewegen sich die Verbindungselemente 122 und 124 innerhalb der Verbindungsebene 130 von selbst wieder zurück in zueinander entgegengesetzten Schließrichtungen 134, so dass in der zweiten Freigabeposition der Haltevorrichtung 36 die zweite Verbindungseinrichtung 46 wieder in die zweite Verbindungsstellung überführt wird. Um die Haltevorrichtung 36 dann wieder mit der Lagereinheit 2 verbinden zu können, kann das freie Ende 94 des Sicherungsabschnitts 90 in die Aufnahme 16 eingeführt werden, bis von den Rastelementen 126 und 128 ausgebildete Anlaufschrägen 136 und 138 mit den Abschnitten 24 der Platte 4 in Eingriff gelangen. Hierdurch werden die Verbindungselemente 122 und 124 in Öffnungsrichtungen 132 aufeinander zu bewegt, so dass die zweite Verbindungseinrichtung 46 in die zweite Lösestellung überführt wird. Die Anlaufschrägen 136 und 138 werden so weit in die Aufnahme 16 eingeführt, dass die Rastelemente 126 und 128 den Abschnitt 24 der Platte 4 rastend hintergreifen und somit die zweite Verbindungseinrichtung 46 wieder die zweite Verbindungsstellung einnimmt.

Die in den Figuren 8 und 9 dargestellte Haltevorrichtung 38 weist einen zu der Haltevorrichtung 36 ähnlichen Aufbau auf. Im Folgenden wird daher nur auf die Unterschiede zwischen den Haltevorrichtungen 36 und 38 eingegangen. Die sich parallel zu der Halteachse 48 erstreckenden Verbindungselemente 122 und 124 der Haltevorrichtung 38 sind nicht an dem freien Ende 94 des Sicherungsabschnitts 90 mit diesem verbunden, sondern über Anbindungen 140 und 142, die benachbart zu dem Verbindungselement 78 vorgesehen sind. Die Rastelemente 126 und 128 sind nicht wie bei der Haltevorrichtung 36 gemäß Figuren 6 und 7 in Form von Vorsprüngen gebildet, sondern werden durch in Richtung auf die Anlagefläche 56 des Grundkörpers 52 weisende Randflächen der Verbindungselemente 122 und 124 gebildet. Die Verbindungselemente 122 und 124 weisen ferner zwei relativ zu der Halteachse 48 in ihrem Verlauf leicht geneigte Randabschnitte 144 und 146 auf, die das Einführen des freien Endes 94 in eine Aufnahme 16 der Platte 4 erleichtern.

Um die Haltevorrichtung 38 relativ zu einer Aufnahme 16 der Platte 4 der Lagereinheit 2 genau positionieren zu können, weist die Haltevorrichtung 38 ausgehend von der Anlagefläche 56 sich parallel zu der Halteachse 48 erstreckende Führungsabschnitte 148 und 150 auf, die in der zweiten Halteposition der Haltevorrichtung 38 jeweils an einem Abschnitt 24 der Platte 4 anliegen.

Die in den Figuren 10 und 11 dargestellte Haltevorrichtung 40 unterscheidet sich von den bisher beschriebenen Haltevorrichtungen 34, 36 und 38 unter anderem dadurch, dass sie eine Halteachse 48 definiert, die nicht im Wesentlichen mittig durch den Grundkörper 52 hindurch verläuft, sondern hierzu seitlich versetzt. Dies hat den Vorteil, dass eine besonders große Sichtfläche 54 entsteht, auf der zueinander eng benachbart bei guter Ablesbarkeit relativ viele Implantat-Daten 76 angezeigt werden können.

Die Implantataufnahme 58 der Haltevorrichtung 40, die von zwei einander gegenüberliegenden Halteelementen 62 sowie dem Anlageabschnitt 60 des Grundkörpers 52 begrenzt ist, ist so weit aus der Mitte des Grundkörpers 52 versetzt, dass der bei den Haltevorrichtungen 34, 36 und 38 vorhandene Freiraum 68 entfällt beziehungsweise durch die Umgebung der Haltevorrichtung 40 gebildet ist.

Die zweite Verbindungseinrichtung 46 der Haltevorrichtung 40 umfasst ähnlich wie bei den Haltevorrichtungen 36 und 38 zwei im Wesentlichen parallel zu der Halteachse 48 verlaufende Verbindungselemente 122 und 124. Im Unterschied zu den Haltevorrichtungen 36 und 38 sind die Verbindungselemente 122 und 124 der Haltevorrichtung 40 von dem Sicherungsabschnitt 90 der Sicherungseinrichtung separat vorgesehen. So ist ein erstes Verbindungselement 122 als U-Schenkel 152 ausgebildet, der sich von der Anlagefläche 56 des Grundkörpers 52 ausgehend parallel zu der Halteachse 48 bis hin zu dem freien Ende 94 des Sicherungsabschnitts 90 erstreckt. Dort geht der U-Schenkel 152 in eine sich parallel zu dem Grundkörper 52 und somit zu der Halteebene 50 erstreckende U-Basis 154 über. Die U-Basis 154 mündet an ihrem dem U-Schenkel 152 gegenüberliegenden Ende an einem U-Schenkel 156, der das Verbindungselement 124 bildet. Die U-Schenkel 152 und 156 und die U-Basis bilden gemeinsam einen U-förmigen Materialabschnitt 158. Der U-Schenkel 156 erstreckt sich von der U-Basis 154 ausgehend in etwa parallel zu der Halteachse 48 in Richtung auf den Grundkörper 52 und - im Bereich eines freien Endes 160, das nicht mit dem Grundkörper 52 verbunden ist - bis hin zu einem Betätigungselement 162. Das Betätigungselement 162 ist in Form eines Griffabschnitts ausgebildet. Dieser Griffabschnitt und die Verbindungselemente 122 und 124 sind entlang der Halteachse 48 gesehen auf einander entgegengesetzten Seiten des Grundkörpers 52 angeordnet.

Um die in den Figuren 10 und 11 dargestellte Haltevorrichtung 40 mit der in Figur 1 dargestellten Lagereinheit 2 zu verbinden, kann das freie Ende 94 der Haltevorrichtung 40 in eine der Aufnahmen 16 der Platte 4 eingesetzt werden. Die zweite Verbindungseinrichtung 46 der Haltevorrichtung 40 wird dabei unter Verformung des Verbindungselements 124 in Richtung auf die Halteachse 48 entsprechend einer Öffnungsrichtung 132 aus der zweiten Verbindungsstellung in die zweite Lösestellung überführt. Wenn die Haltevorrichtung 40 so weit in eine der Aufnahmen 16 eingeführt ist, dass die Rastelemente 126 und 128 zugeordnete Abschnitte 24 der Aufnahme 16 rastend hintergreifen können, federt das Verbindungselement 124 entsprechend einer Schließrichtung 134 zurück in die in Figur 10 dargestellte Stellung, so dass die zweite Verbindungseinrichtung 46 in die zweite Verbindungsstellung überführt wird.

Um die zweite Verbindungseinrichtung 46 aus der zweiten Verbindungsstellung in die zweite Lösestellung zu überführen, kann das Betätigungselement 162 entsprechend der Öffnungsrichtung 132, die senkrecht zu der Halteachse 48 verläuft, betätigt werden. Hierbei wird das Verbindungselement 124 beziehungsweise der U-Schenkel 156 innerhalb einer Verbindungsebene 130 verformt, so dass das Rastelement 128 außer Eingriff mit dem zugeordneten Abschnitt 24 der Platte 4 gebracht werden kann, wodurch die zweite Verbindungseinrichtung 46 in die zweite Lösestellung überführt wird. Auf diese Weise kann die Haltevorrichtung 40 bei ihrer Bewegung entsprechend einer zweiten Handhabungsrichtung 118, die parallel zu der Halteachse 48 verläuft, von der Lagereinheit 2 gelöst werden.

Die U-Schenkel 152 und 156 der Haltevorrichtung 40 sind relativ zu der Halteachse 48 auf einander gegenüberliegenden Seiten angeordnet. Dies hat den Vorteil, dass ein über die erste Verbindungseinrichtung 44 mit der Haltevorrichtung 40 verbundenes Implantat 42 nicht nur durch den Sicherungsabschnitt 90, sondern auch mithilfe des U-förmigen Materialabschnitts 158 vor mechanischen Einflüssen geschützt ist. Dabei schirmt die U-Basis 154 das Implantat 42 gegenüber der in Figur 1 dargestellten Aufstellfläche 14 ab, wenn die Haltevorrichtung 40 mit der Lagereinheit 2 verbunden ist.

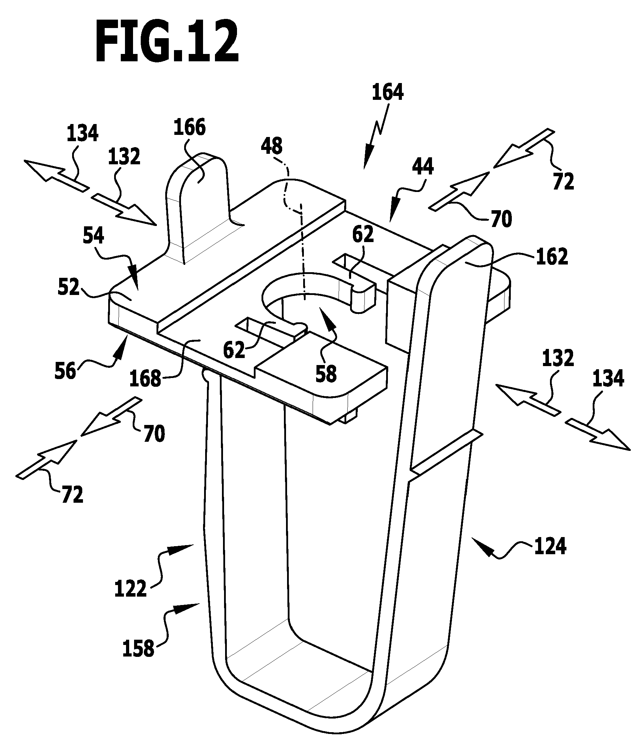

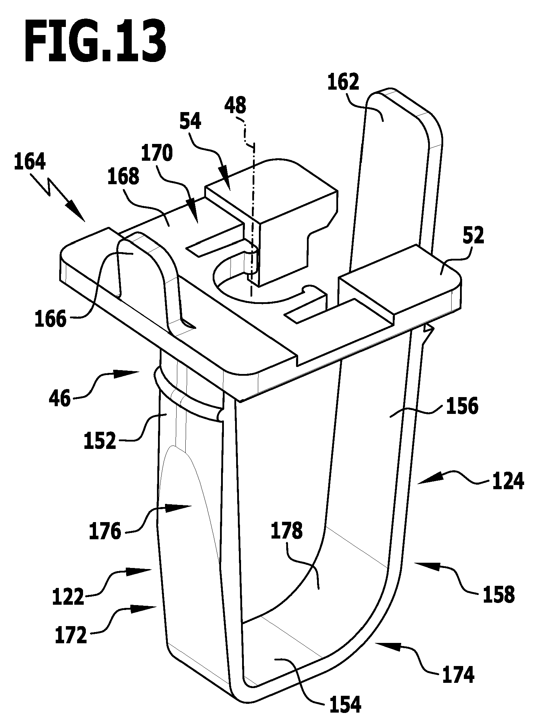

In den Figuren 12 und 13 ist eine weitere Haltevorrichtung 164 dargestellt. Diese umfasst ebenfalls einen U-förmigen Materialabschnitt 158. Im Unterschied zu der Haltevorrichtung 40 gemäß Figuren 10 und 11 verläuft die Halteachse 48 der Haltevorrichtung 164 in etwa mittig durch den Grundkörper 52 hindurch. Darüber hinaus sind die Halteelemente 62 der ersten Verbindungseinrichtung 44 derart orientiert, dass ihre Öffnungs- und Schließrichtungen 70 und 72 nicht wie bei der Haltevorrichtung 40 parallel zu den Öffnungsrichtungen 132 und Schließrichtungen 134 der Verbindungselemente 122 und 124 der zweiten Verbindungseinrichtung 46 verlaufen, sondern zueinander senkrecht.

Die Haltevorrichtung 164 weist ferner ein Betätigungselement 166 auf, das fest mit dem Grundkörper 52 verbunden ist, von der Sichtfläche 54 abragt und sich in etwa parallel zu der Halteachse 48 erstreckt. Die Betätigungselemente 162 und 166 sind auf einander gegenüberliegenden Seiten der Halteachse 48 angeordnet und können relativ zueinander in den genannten Öffnungsrichtungen 132 beziehungsweise Schließrichtungen 134 bewegt werden, um die zweite Verbindungseinrichtung 46 aus der zweiten Verbindungsstellung in die zweite Lösestellung zu überführen.

Der Grundkörper 52 weist in einem mittleren Abschnitt 168 eine reduzierte Materialstärke auf. Der mittlere Abschnitt 168 weist eine sich parallel zu der Sichtfläche 54 des Grundkörpers 52 erstreckende Fläche 170 auf, die geringer zu der Anlagefläche 56 des Grundkörpers 52 beabstandet ist als die Sichtfläche 54. Hierdurch ist die Fläche 170 gegenüber der Sichtfläche 54 zurückversetzt. Wenn die Haltevorrichtung 164 mithilfe ihrer ersten Verbindungseinrichtung 44 mit einem Implantat 42 verbunden ist, erstreckt sich ein Implantatabschnitt 100 (vgl. Figur 3) nicht oder nur geringfügig über die Sichtfläche 54 hinaus. Dies hat zur Folge, dass die Haltevorrichtung 164 und ein Implantat 42 gemeinsam einer Beschriftungseinrichtung zugeführt werden kann, mit der Implantat-Daten 76 auf die Sichtfläche 54 der Haltevorrichtung 164 aufgebracht werden können, beispielsweise durch Aufdrucken.

Die in den Figuren 12 und 13 dargestellte Haltevorrichtung 164 unterscheidet sich ferner von der in den Figuren 10 und 11 dargestellten Haltevorrichtung 40 dadurch, dass ihr U-förmiger Materialabschnitt 158 Einführhilfen 172 und 174 aufweist. Die Einführhilfe 172 umfasst eine leicht winklig zu der Halteachse 48 verlaufende Einführfläche 176. Die Einführhilfe 174 ist durch einen teilzylindrischen Übergangsabschnitt zwischen der U-Basis 154 und dem U-Schenkel 156 gebildet. Die Einführhilfen 172 und 174 erleichtern das Einführen der Haltevorrichtung 164 in eine Aufnahme 16 der Lagereinheit 2.

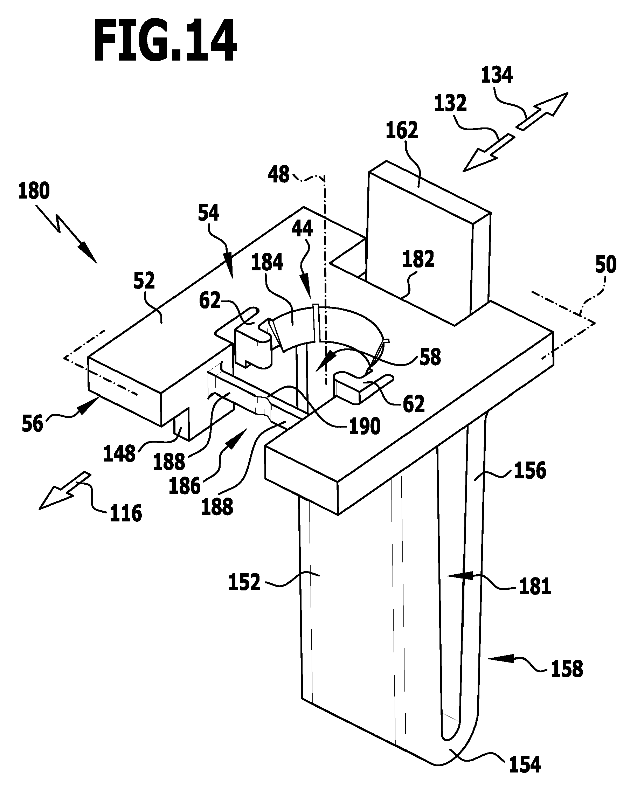

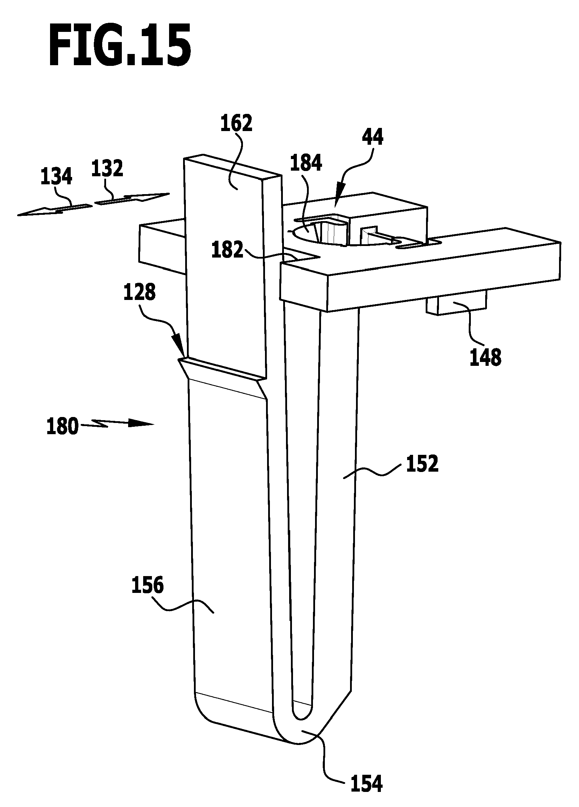

In den Figuren 14 und 15 ist eine weitere Haltevorrichtung 180 dargestellt. Diese unterscheidet sich von den Haltevorrichtungen 40 und 164 dadurch, dass ihr U-förmiger Materialabschnitt 158 derart ausgebildet ist, dass die U-Schenkel 152 und 156 zueinander direkt benachbart sind. Die Halteachse 48 der Haltevorrichtung 180 verläuft außerhalb eines zwischen den U-Schenkeln 152 und 156 ausgebildeten Raums 181.

Der Grundkörper 52 der Haltevorrichtung 180 weist eine sich innerhalb der Halteebene 50 erstreckende Vertiefung 182 auf. Diese schafft einen Bewegungsraum für den U-Schenkel 156, der mithilfe des Betätigungselements 162 in Öffnungsrichtung 132 oder in Schließrichtung 134 bewegt werden kann.

Die Implantataufnahme 58 der ersten Verbindungseinrichtung 44 der Haltevorrichtung 180 unterscheidet sich von den bisher beschriebenen Implantataufnahmen 58 der Haltevorrichtungen 34, 36, 38, 40, 164 dadurch, dass die einander gegenüberliegenden Halteelemente 62 vergleichsweise kurz sind. Anstelle eines starren Anlageabschnitts 60 (vgl. Figur 2) weist die Implantataufnahme 58 der Haltevorrichtung 180 kreissegmentförmige Anlageelemente 184 auf. Diese sind lappenförmig ausgebildet und auf Höhe der Sichtfläche 54 des Grundkörpers 52 mit diesem verbunden. Die Anlageelemente 184 erstrecken sich ausgehend von der Sichtfläche 54 winklig in Richtung auf die Halteachse 48.

Wenn ein Implantat 42 mithilfe der ersten Verbindungseinrichtung 44 mit der Haltevorrichtung 180 verbunden ist, liegen die Anlageelemente 184 unter Vorspannung an dem Implantatabschnitt 102 (vgl. Figur 3) an. Hierdurch ist das Implantat 42 spielfrei mit der Haltevorrichtung 180 verbunden.

Die Haltevorrichtung 180 umfasst eine Anzeigevorrichtung 186, mit der angezeigt werden kann, ob die erste Verbindungseinrichtung 44 der Haltevorrichtung 180 zumindest einmalig aus der ersten Verbindungsstellung in die erste Lösestellung überführt wurde. Die Anzeigevorrichtung 186 umfasst zwei bändchenförmige Anzeigeelemente 188, die sich innerhalb der Halteebene 50 benachbart zu der Implantataufnahme 58 erstrecken. Die Anzeigeelemente 188 sind über einen Verbindungsabschnitt 190 miteinander verbunden, der eine Sollbruchstelle bildet.

Wenn ein mit der Haltevorrichtung 180 verbundenes Implantat 42 entsprechend einer ersten Handhabungsrichtung 116 in zu der Halteachse 48 senkrechter Richtung aus einer ersten Halteposition in eine erste Freigabeposition überführt wird, bewirkt dies eine Zerstörung der Verbindung zwischen den Anzeigeelementen 188. Hierdurch ist eindeutig feststellbar, dass von der Haltevorrichtung 180 bereits ein Implantat 42 entfernt wurde. Hierdurch kann auch eine unbeabsichtigte Wiederverwendung der Haltevorrichtung 180 ausgeschlossen werden.

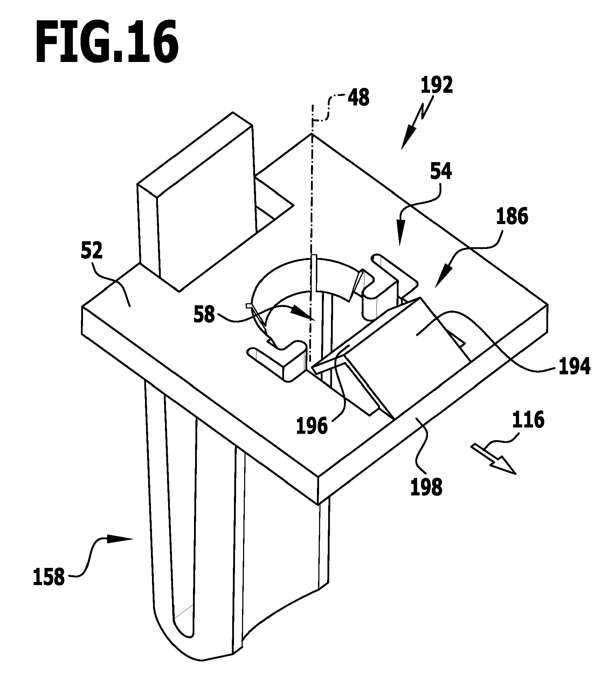

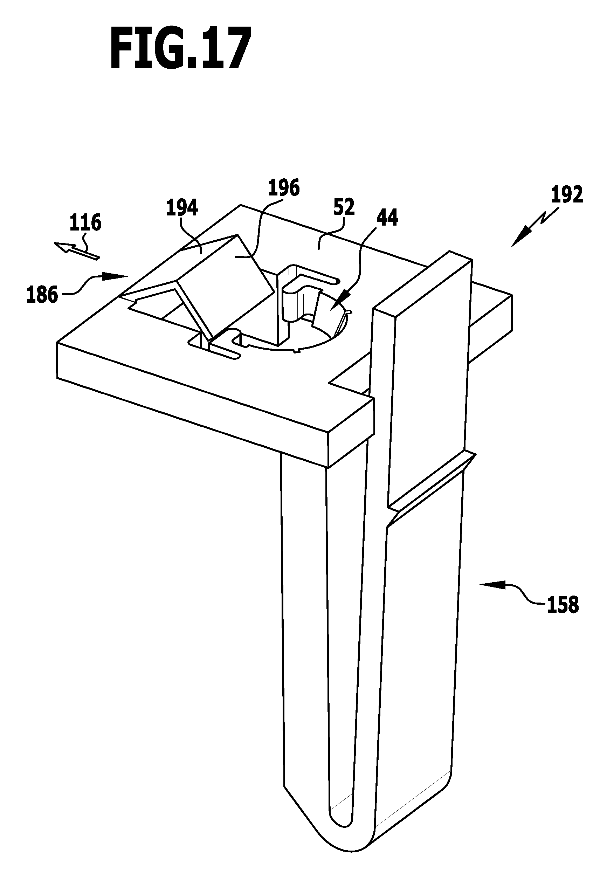

In den Figuren 16 und 17 ist eine weitere Haltevorrichtung 192 dargestellt, die sich von der Haltevorrichtung 180 gemäß Figuren 14 und 15 lediglich in ihrer Ausgestaltung der Anzeigevorrichtung 186 unterscheidet. Die Anzeigevorrichtung 186 der Haltevorrichtung 192 umfasst zwei zueinander in einem stumpfen Winkel angeordnete Elementabschnitte 194 und 196. Der Elementabschnitt 194 ist an einem geschlossenen Rand 198 des Grundkörpers 52 angelenkt und erstreckt sich in einem spitzen Winkel relativ zu der Sichtfläche 54 des Grundkörpers 52 in Richtung auf die Halteachse 48. Der Elementabschnitt 196 erstreckt sich ausgehend von dem der Halteachse 48 zugewandten Ende des Elementabschnitts 194 in Richtung auf die Halteachse 48 bis hin benachbart zu der Implantataufnahme 58.

Wenn ein an der Haltevorrichtung 192 gehaltenes Implantat 42 in einer ersten Handhabungsrichtung 116 aus einer ersten Halteposition in eine erste Freigabeposition überführt wird, verformen sich die Elementabschnitte 194 und 196 dauerhaft, so dass angezeigt werden kann, dass die erste Verbindungseinrichtung 44 der Haltevorrichtung 192 aus der ersten Verbindungsstellung in die erste Lösestellung überführt wurde.

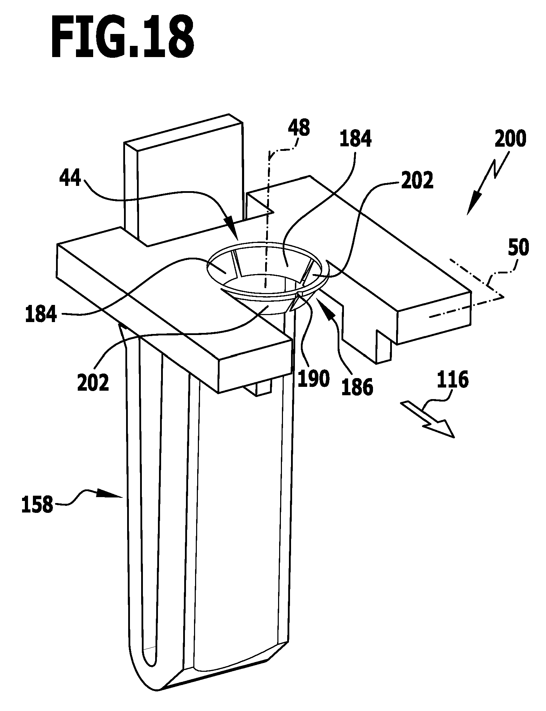

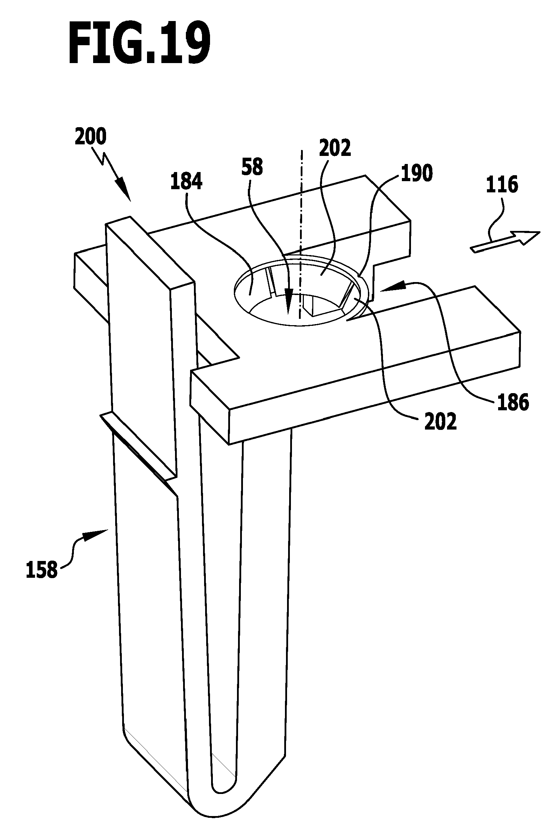

Die in den Figuren 18 und 19 dargestellte Haltevorrichtung 200 unterscheidet sich von den Haltevorrichtungen 180 und 192 in seiner Ausgestaltung der ersten Verbindungseinrichtung 44. Diese umfasst ebenfalls kreissegmentförmige Anlageelemente 184, jedoch keine im Wesentlichen zungenförmigen Halteelemente 62, sondern kreissegmentförmige Halteelemente 202, die gemeinsam mit den Anlageelementen 184 eine insgesamt umfangsseitig geschlossene Implantataufnahme 58 begrenzen, die sich innerhalb einer Halteebene 50 erstreckt. Die Halteelemente 202 sind über einen Verbindungsabschnitt 190 miteinander verbunden, der eine Sollbruchstelle bildet.

Ein Implantat 42 kann in die Haltevorrichtung 200 eingesetzt werden, indem es mit seinem Implantatabschnitt 104 (vgl. Figur 3) voran in Richtung der Halteachse 48 in die Implantataufnahme 58 eingesetzt wird. Hierbei werden die Anlageelemente 184 und die Halteelemente 202 durch das Außengewinde 106 nach radial außen verformt, so dass der Implantatabschnitt 104 vollständig in die Implantataufnahme eingeführt werden kann, bis der Implantatabschnitt 102 auf Höhe der Anlageelemente 184 und der Halteelemente 202 angeordnet ist und die Anlageelemente 184 und die Halteelemente 202 sich wieder nach radial innen zurückstellen können. Dann nimmt die Verbindungseinrichtung 44 die erste Verbindungsstellung ein.

Um das Implantat 42 von der Haltevorrichtung 200 zu lösen, kann die erste Verbindungseinrichtung 44 aus der ersten Verbindungsstellung in die erste Lösestellung gebracht werden, indem das Implantat 42 in mit 116 bezeichneter erster Handhabungsrichtung aus der Implantataufnahme 58 heraus bewegt wird. Hierbei wird der Verbindungsabschnitt 190 zwischen den Halteelementen 202 zerstört. Hierdurch kann angezeigt werden, dass an der Haltevorrichtung 200 bereits ein Implantat 42 gehalten war, so dass eine unbeabsichtigte Wiederverwendung der Haltevorrichtung 200 ausgeschlossen werden kann. Die Haltevorrichtung 200 umfasst also ebenfalls eine Anzeigevorrichtung 186. Bei dieser Anzeigevorrichtung sind die Anzeigeelemente durch die Halteelemente 202 gebildet.

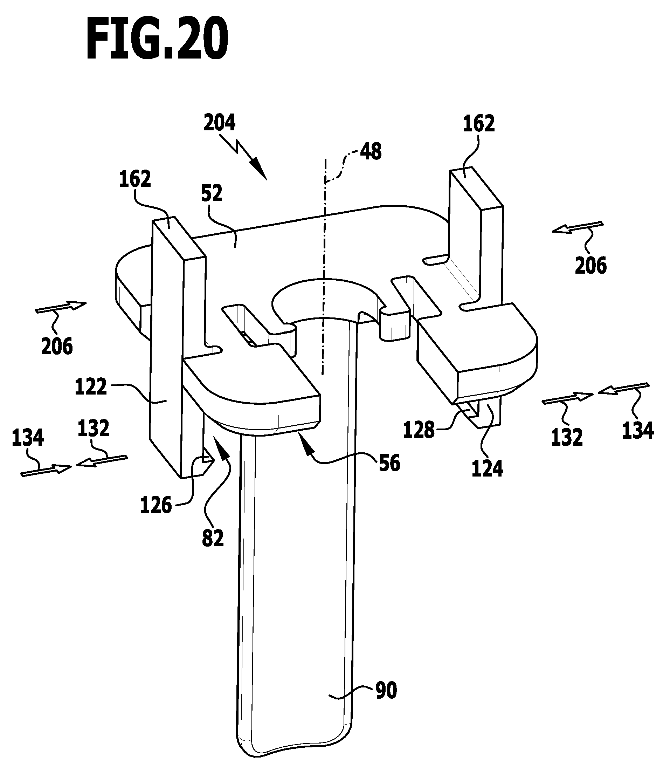

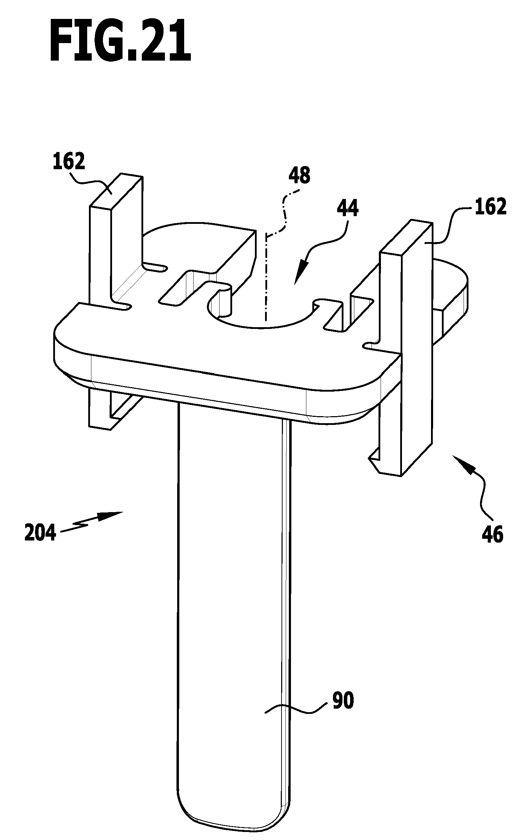

In den Figuren 20 und 21 ist eine weitere Haltevorrichtung 204 dargestellt. Deren erste Verbindungseinrichtung 44 weist einen Aufbau auf, der beispielsweise dem Aufbau der ersten Verbindungseinrichtung 44 der Haltevorrichtung 34 gemäß Figur 2 entspricht. Die Haltevorrichtung 204 weist ebenfalls einen Sicherungsabschnitt 90 auf, der in seinem Aufbau dem Sicherungsabschnitt 90 der Haltevorrichtung 40 gemäß Figuren 10 und 11 entspricht.

Hingegen unterscheidet sich die zweite Verbindungseinrichtung 46 der Haltevorrichtung 204 von den zweiten Verbindungseinrichtungen 46 der Haltevorrichtungen 34, 36, 38, 40, 164, 180, 192, 200 dadurch, dass die Rastelemente 126 und 128 auf der der Halteachse 48 zugewandten Seite der Verbindungselemente 122 und 124 angeordnet sind. Dies bewirkt eine Umkehr der entsprechenden Öffnungsrichtungen 132 und der Schließrichtungen 134. Außerdem ist ein zwischen den Rastelementen 126 und 128 und der Anlagefläche 56 des Grundkörpers 52 ausgebildeter Hinterschnittbereich 82 zwischen den Verbindungselementen 122 und 124 ausgebildet und nicht auf einander entgegengesetzten Seiten.

Die Haltevorrichtung 204 kann mit der in Figur 1 dargestellten Lagereinheit 2 verbunden werden, indem der Sicherungsabschnitt 90 der Haltevorrichtung 204 in eine Aufnahme 16 der Platte 4 eingetaucht wird, bis die Rastelemente 126 und 128 in die Lagerbereiche 32 der Lagerelemente 26 eingreifen. Die zweite Verbindungseinrichtung 46 der Haltevorrichtung 204 nimmt dann die zweite Verbindungsstellung ein.

Um die zweite Verbindungseinrichtung 46 der Haltevorrichtung 204 in die zweite Lösestellung zu überführen, können die Betätigungselemente 162 der Verbindungselemente 122 und 124 in zueinander entgegensetzten Betätigungsrichtungen 206 aufeinander zu bewegt werden, so dass die Rastelemente 126 und 128 in Öffnungsrichtung 132 voneinander weg bewegt werden und außer Eingriff mit den Lagerbereichen 32 der Lagerelemente 26 gebracht werden.

## Patentansprüche

1. Haltevorrichtung (34, 36, 38, 40, 164, 180, 192, 200, 204) für ein Implantat (42), mit einer ersten Verbindungseinrichtung (44) zum lösbaren Verbinden der Haltevorrichtung (34, 36, 38, 40, 164, 180, 192, 200, 204) mit dem Implantat (42), wobei die erste Verbindungseinrichtung (44) aus einer ersten Verbindungsstellung, in der ein Implantat (42) mit der Haltevorrichtung (34, 36, 38, 40, 164, 180, 192, 200, 204) verbindbar oder verbunden ist, in eine erste Lösestellung, in der die Haltevorrichtung (34, 36, 38, 40, 164, 180, 192, 200, 204) das Implantat (42) freigibt, überführbar ist, **dadurch gekennzeichnet, dass** die Haltevorrichtung eine Anzeigevorrichtung (186) aufweist, die eine zumindest einmalige Überführung der ersten Verbindungseinrichtung (44) aus der ersten Verbindungsstellung in die erste Lösestellung anzeigt.

2. Haltevorrichtung (34, 36, 38, 40, 164, 180, 192, 200, 204) nach Anspruch 1, **dadurch gekennzeichnet, dass** sie eine zweite Verbindungseinrichtung (46) zum lösbaren Verbinden der Haltevorrichtung (34, 36, 38, 40, 164, 180, 192, 200, 204) mit einer Lagereinheit (2) aufweist.

3. Haltevorrichtung (34, 36, 38, 40, 164, 180, 192, 200, 204) nach Anspruch 2, **dadurch gekennzeichnet, dass** die erste Verbindungseinrichtung (44) und die zweite Verbindungseinrichtung (46) voneinander unabhängig betätigbar sind.

4. Haltevorrichtung (34, 36, 38, 40, 164, 180, 192, 200, 204) nach Anspruch 3, **dadurch gekennzeichnet, dass** die erste Verbindungseinrichtung (44) derart ausgebildet ist, dass zur Überführung der ersten Verbindungseinrichtung (44) aus der ersten Verbindungsstellung in die erste Lösestellung eine erste Lösekraft erforderlich ist.

5. Haltevorrichtung (34, 36, 38, 40, 164, 180, 192, 200, 204) nach Anspruch 3 oder 4, **dadurch gekennzeichnet, dass** sie eine erste Rückstelleinrichtung (73) umfasst, die die erste Verbindungseinrichtung (44) aus der ersten Lösestellung in die erste Verbindungsstellung überführt.

6. Haltevorrichtung (34, 36, 38, 40, 164, 180, 192, 200, 204) nach einem der Ansprüche 2 bis 5, **dadurch gekennzeichnet, dass** die zweite Verbindungseinrichtung (46) aus einer zweiten Verbindungsstellung, in der die Haltevorrichtung (34, 36, 38, 40, 164, 180, 192, 200, 204) mit der Lagereinheit (2) verbindbar oder verbunden ist, in eine zweite Lösestellung, in der die Haltevorrichtung (34, 36, 38, 40, 164, 180, 192, 200, 204) von der Lagereinheit (2) lösbar ist, überführbar ist.

7. Haltevorrichtung (34, 36, 38, 40, 164, 180, 192, 200, 204) nach Anspruch 6, **dadurch gekennzeichnet, dass** die zweite Verbindungseinrichtung (46) derart ausgebildet ist, dass zur Überführung der zweiten Verbindungseinrichtung (46) aus der zweiten Verbindungsstellung in die zweite Lösestellung eine zweite Lösekraft erforderlich ist.

8. Haltevorrichtung (34, 36, 38, 40, 164, 180, 192, 200, 204) nach Anspruch 6 oder 7, **dadurch gekennzeichnet, dass** sie eine zweite Rückstelleinrichtung (81) umfasst, die die zweite Verbindungseinrichtung (46) aus der zweiten Lösestellung in die zweite Verbindungsstellung überführt.

9. Haltevorrichtung (34, 36, 38, 40, 164, 180, 192, 200, 204) nach Anspruch 4 und 7 oder nach den Ansprüchen 4, 7 und 8, **dadurch gekennzeichnet, dass** sich die erste Lösekraft und die zweite Lösekraft nach Betrag und/oder Richtung voneinander unterscheiden.

10. Haltevorrichtung (34, 36, 38, 40, 164, 180, 192, 200, 204) nach den Ansprüchen 4 und 7 oder nach den Ansprüchen 4, 7 und 8 oder nach Anspruch 9, **dadurch gekennzeichnet, dass** die erste Lösekraft und die zweite Lösekraft voneinander linear unabhängig sind.

11. Haltevorrichtung (34, 36, 38, 40, 164, 180, 192, 200, 204) nach den Ansprüchen 4 und 7 oder nach den Ansprüchen 4, 7 und 8 oder nach einem der Ansprüche 9 oder 10, **dadurch gekennzeichnet, dass** die erste Lösekraft kleiner ist als die zweite Lösekraft.

12. Haltevorrichtung (34, 36, 38, 40, 164, 180, 192, 200, 204) nach einem der voranstehenden Ansprüche, **dadurch gekennzeichnet, dass** die erste Verbindungseinrichtung (44) derart ausgebildet ist, dass das Implantat (42) bei einer Bewegung aus einer ersten Halteposition, in der das Implantat (42) mit der Haltevorrichtung (34, 36, 38, 40, 164, 180, 192, 200, 204) verbunden ist, in eine erste Freigabeposition, in der das Implantat (42) von der Haltevorrichtung (34, 36, 38, 40, 164, 180, 192, 200, 204) gelöst ist, in einer ersten Handhabungsrichtung (116) handhabbar ist.

13. Haltevorrichtung (34, 36, 38, 40, 164, 180, 192, 200, 204) nach einem der Ansprüche 2 bis 11 oder nach den Ansprüchen 2 und 12, **dadurch gekennzeichnet, dass** die zweite Verbindungseinrichtung (46) derart ausgebildet ist, dass die Haltevorrichtung (34, 36, 38, 40, 164, 180, 192, 200, 204) bei einer Bewegung aus einer zweiten Halteposition, in der die Haltevorrichtung (34, 36, 38, 40, 164, 180, 192, 200, 204) mit der Lagereinheit (2) verbunden ist, in eine zweite Freigabeposition, in der die Haltevorrichtung (34, 36, 38, 40, 164, 180, 192, 200, 204) von der Lagereinheit (2) gelöst ist, in einer zweiten Handhabungsrichtung (118) handhabbar ist.

14. Haltevorrichtung (34, 36, 38, 40, 164, 180, 192, 200, 204) nach Anspruch 12 und 13, **dadurch gekennzeichnet, dass** die erste Handhabungsrichtung (116) und die zweite Handhabungsrichtung (118) voneinander linear unabhängig sind.

15. Haltevorrichtung (34, 36, 38, 40, 164, 180, 192, 200, 204) nach einem der Ansprüche 3 bis 13, **dadurch gekennzeichnet, dass** die Haltevorrichtung (34, 36, 38, 40, 164, 180, 192, 200, 204) eine Halteachse (48) definiert, die die Position und/oder die Lage des Implantats (42) vorgibt, wenn dieses mit der Haltevorrichtung (34, 36, 38, 40, 164, 180, 192, 200, 204) verbunden ist.

16. Haltevorrichtung (34, 36, 38, 40, 164, 180, 192, 200, 204) nach Anspruch 15 und einem der Ansprüche 13 oder 14, **dadurch gekennzeichnet, dass** die Halteachse (48) und die zweite Handhabungsrichtung (118) zueinander parallel oder im Wesentlichen parallel sind.

17. Haltevorrichtung (34, 36, 38, 40, 164, 180, 192, 200, 204) nach einem der Ansprüche 3 bis 16, **dadurch gekennzeichnet, dass** die erste Verbindungseinrichtung (44) mindestens ein Halteelement (62, 202) umfasst, das ausgebildet ist, in der ersten Verbindungsstellung der ersten Verbindungseinrichtung (44) das Implantat (42) mit der Haltevorrichtung (34, 36, 38, 40, 164, 180, 192, 200, 204) zu verbinden.

18. Haltevorrichtung (34, 36, 38, 40, 164, 180, 192, 200, 204) nach Anspruch 3 und 17, **dadurch gekennzeichnet, dass** mindestens ein Halteelement (62, 202) zur Ausbildung der ersten Rückstelleinrichtung (73) bei Überführung der ersten Verbindungseinrichtung (44) aus der ersten Verbindungsstellung in die erste Lösestellung eine erste Rückstellkraft aufbaut, mit der die erste Verbindungseinrichtung (44) zurück in die erste Verbindungsstellung überführbar ist.

19. Haltevorrichtung (34, 36, 38, 40, 164, 180, 192, 200, 204) nach Anspruch 17 oder 18, **dadurch gekennzeichnet, dass** das mindestens eine Halteelement (62, 202) eine Implantataufnahme (58) zur Aufnahme des Implantats (42) begrenzt.

20. Haltevorrichtung (34, 36, 38, 40, 164, 180, 192, 204) nach Anspruch 19, **dadurch gekennzeichnet, dass** die Implantataufnahme (58) einen Hinterschnitt aufweist.

21. Haltevorrichtung (180, 192, 200) nach einem der voranstehenden Ansprüche, **dadurch gekennzeichnet, dass** die Anzeigevorrichtung (186) mindestens ein Anzeigeelement (188) umfasst, das bei einer Überführung der ersten Verbindungseinrichtung (44) aus der ersten Verbindungsstellung in die erste Lösestellung zerstörbar und/oder plastisch verformbar ist.

22. Haltevorrichtung (200) nach Anspruch 21, **dadurch gekennzeichnet, dass** das mindestens eine Anzeigeelement (188) durch ein Halteelement (202) gebildet ist.

23. Haltevorrichtung (34, 36, 38, 40, 164, 180, 192, 200, 204) nach einem der voranstehenden Ansprüche, **dadurch gekennzeichnet, dass** sie einen plattenförmigen Grundkörper (52) umfasst.

24. Haltevorrichtung (34, 36, 38, 40, 164, 180, 192, 200, 204) nach Anspruch 19, 22 und 23, **dadurch gekennzeichnet, dass** die erste Verbindungseinrichtung mindestens eine Anlageelement umfasst, das in der ersten Verbindungsstellung der ersten Verbindungseinrichtung unter Vorspannung an das Implantat anlegbar ist und dass das mindestens eine Halteelement (62, 202) und/oder das mindestens eine Anlageelement (184) und/oder das mindestens eine Anzeigeelement (188) mit dem Grundkörper (52) einstückig ausgebildet ist oder sind.

25. Haltevorrichtung (34, 36, 38, 40, 164, 180, 192, 200, 204) nach einem der voranstehenden Ansprüche, **dadurch gekennzeichnet, dass** sie einen Datenspeicher (74) zur Speicherung von Implantat-Daten (76) umfasst.

26. Haltevorrichtung (34, 36, 38, 40, 164, 180, 192, 200, 204) nach einem der Ansprüche 2-11, 13-14, 16, 18 oder nach einem der Ansprüche 12, 15, 17, 19-25 und Anspruch 2, **dadurch gekennzeichnet, dass** die zweite Verbindungseinrichtung (46) mindestens ein Verbindungselement (78, 122, 124) umfasst, das ausgebildet ist, in der zweiten Verbindungsstellung der zweiten Verbindungseinrichtung (46) die Haltevorrichtung (34, 36, 38, 40, 164, 180, 192, 200, 204) mit der Lagereinheit (2) zu verbinden.

27. Haltevorrichtung (34, 36, 38, 40, 164, 180, 192, 200, 204) nach Anspruch 26, **dadurch gekennzeichnet, dass** das mindestens eine Verbindungselement (78, 122, 124) zur Ausbildung einer Rastverbindung mindestens ein Rastelement (80, 126, 128) umfasst oder als Rastelement (80, 126, 128) ausgebildet ist, wobei das Rastelement (80, 126, 128) in der zweiten Verbindungsstellung der zweiten Verbindungseinrichtung (46) mit der Lagereinheit (2) rastend in Eingriff steht oder in Eingriff bringbar ist.

28. Haltevorrichtung (34, 36, 38, 40, 164, 180, 192, 200, 204) nach Anspruch 26 oder 27, **dadurch gekennzeichnet, dass** das mindestens eine Verbindungselement (78, 122, 124) innerhalb einer Verbindungsebene (130) beweglich und/oder verformbar ist.

29. Haltevorrichtung (34, 36, 38, 40, 164, 180, 192, 200, 204) nach Anspruch 6 und einem der Ansprüche 26 bis 28, **dadurch gekennzeichnet, dass** das mindestens eine Verbindungselement (78, 122, 124) in der zweiten Verbindungsstellung der zweiten Verbindungseinrichtung (46) einen Hinterschnittbereich (82) zur Aufnahme eines Abschnitts (24, 30) der Lagereinheit (2) begrenzt.

30. Haltevorrichtung (40, 164, 180, 192, 200) nach einem der Ansprüche 26 bis 29, **dadurch gekennzeichnet, dass** die zweite Verbindungseinrichtung (46) mindestens einen im Wesentlichen U-förmigen Materialabschnitt (158) umfasst, der zwei sich parallel oder im Wesentlichen parallel zu der Halteachse (48) erstreckende U-Schenkel (152, 156) aufweist, die über eine U-Basis (154) miteinander verbunden sind.

31. Haltevorrichtung (40, 164, 180, 192, 200, 204) nach Anspruch 6 und einem der Ansprüche 7 bis 30, **dadurch gekennzeichnet, dass** die zweite Verbindungseinrichtung (46) mindestens ein Betätigungselement (162, 166) zum Überführen der zweiten Verbindungseinrichtung (46) aus der zweiten Verbindungsstellung in die zweite Lösestellung aufweist.

32. Haltevorrichtung (34, 36, 38, 40, 164, 180, 192, 200, 204) nach einem der voranstehenden Ansprüche, **dadurch gekennzeichnet, dass** sie mindestens eine Sicherungseinrichtung (88) umfasst, die ein Implantieren des Implantats (42) verhindert, wenn das Implantat (42) mit der Haltevorrichtung (34, 36, 38, 40, 164, 180, 192, 200, 204) verbunden ist.

33. Haltevorrichtung (34, 36, 38, 40, 164, 180, 192, 200, 204) nach einem der voranstehenden Ansprüche, **dadurch gekennzeichnet, dass** sie eine Führungseinrichtung (84) aufweist, mit der die Haltevorrichtung (34, 36, 38, 40, 164, 180, 192, 200, 204) relativ zu der Lagereinheit (2) positionierbar ist.

34. Haltevorrichtung (34, 36, 38, 40, 164, 180, 192, 200, 204) nach einem der voranstehenden Ansprüche mit mindestens einem Implantat (42).

35. Lagereinheit (2) zum Aufnehmen und/oder Festlegen mindestens einer Haltevorrichtung (34, 36, 38, 40, 164, 180, 192, 200, 204) für ein Implantat (42), welche Lagereinheit eine Haltevorrichtung (34, 36, 38, 40, 164, 180, 192, 200, 204) nach einem der voranstehenden Ansprüche umfasst.

36. Lagereinheit (2) nach Anspruch 35, **dadurch gekennzeichnet, dass** sie zum Aufnehmen und/oder Festlegen mindestens einer Haltevorrichtung (34, 36, 38, 40, 164, 180, 192, 200, 204) mindestens eine Aufnahme (16) umfasst.

## Claims

1. Holding device (34, 36, 38, 40, 164, 180, 192, 200, 204) for an implant (42), comprising a first connecting device (44) for releasably connecting the holding device (34, 36, 38, 40, 164, 180, 192, 200, 204) and the implant (42), wherein the first connecting device (44) is transferable from a first connecting position, an implant (42) being connectable or connected to the holding device (34, 36, 38, 40, 164, 180, 192, 200, 204) in said position, into a first release position, the holding device (34, 36, 38, 40, 164, 180, 192, 200, 204) releasing the implant (42) in said position, **characterized in that** the holding device has an indicating device (186) indicating an at least one-time transfer of the first connecting device (44) from the first connecting position into the first release position.

2. Holding device (34, 36, 38, 40, 164, 180, 192, 200, 204) as defined in claim 1, **characterized in that** it has a second connecting device (46) for releasably connecting the holding device (34, 36, 38, 40, 164, 180, 192, 200, 204) and a storage unit (2).

3. Holding device (34, 36, 38, 40, 164, 180, 192, 200, 204) as defined in claim 2, **characterized in that** the first connecting device (44) and the second connecting device (46) are actuatable independently of one another.

4. Holding device (34, 36, 38, 40, 164, 180, 192, 200, 204) as defined in claim 3, **characterized in that** the first connecting device (44) is designed in such a manner that a first releasing force is required to transfer the first connecting device (44) from the first connecting position into the first release position.

5. Holding device (34, 36, 38, 40, 164, 180, 192, 200, 204) as defined in claim 3 or 4, **characterized in that** it comprises a first restoring device (73) transferring the first connecting device (44) from the first release position into the first connecting position.

6. Holding device (34, 36, 38, 40, 164, 180, 192, 200, 204) as defined in any one of claims 2 to 5, **characterized in that** the second connecting device (46) is transferable from a second connecting position, the holding device (34, 36, 38, 40, 164, 180, 192, 200, 204) being connectable or connected to the storage unit (2) in said position, into a second release position, the holding device (34, 36, 38, 40, 164, 180, 192, 200, 204) being releasable from the storage unit (2) in said position.

7. Holding device (34, 36, 38, 40, 164, 180, 192, 200, 204) as defined in claim 6, **characterized in that** the second connecting device (46) is designed in such a manner that a second releasing force is required to transfer the second connecting device (46) from the second connecting position into the second release position.

8. Holding device (34, 36, 38, 40, 164, 180, 192, 200, 204) as defined in claim 6 or 7, **characterized in that** it comprises a second restoring device (81) transferring the second connecting device (46) from the second release position into the second connecting position.

9. Holding device (34, 36, 38, 40, 164, 180, 192, 200, 204) as defined in claims 4 and 7 or in claims 4, 7 and 8, **characterized in that** the first releasing force and the second releasing force differ from one another according to amount and/or direction.

10. Holding device (34, 36, 38, 40, 164, 180, 192, 200, 204) as defined in claims 4 and 7 or in claims 4, 7 and 8 or in claim 9, **characterized in that** the first releasing force and the second releasing force are linearly independent of one another.

11. Holding device (34, 36, 38, 40, 164, 180, 192, 200, 204) as defined in claims 4 and 7 or in claims 4, 7 and 8 or in any one of claims 9 or 10, **characterized in that** the first releasing force is smaller than the second releasing force.

12. Holding device (34, 36, 38, 40, 164, 180, 192, 200, 204) as defined in any one of the preceding claims, **characterized in that** the first connecting device (44) is designed in such a manner that the implant (42) is adapted to be handled in a first handling direction (116) during movement from a first holding position, the implant (42) being connected to the holding device (34, 36, 38, 40, 164, 180, 192, 200, 204) in said position, into a first release position, the implant (42) being released from the holding device (34, 36, 38, 40, 164, 180, 192, 200, 204) in said position.

13. Holding device (34, 36, 38, 40, 164, 180, 192, 200, 204) as defined in any one of claims 2 to 11 or in claims 2 and 12, **characterized in that** the second connecting device (46) is designed in such a manner that the holding device (34, 36, 38, 40, 164, 180, 192, 200, 204) is adapted to be handled in a second handling direction (118) during movement from a second holding position, the holding device (34, 36, 38, 40, 164, 180, 192, 200, 204) being connected to the storage unit (2) in said position, into a second release position, the holding device (34, 36, 38, 40, 164, 180, 192, 200, 204) being released from the storage unit (2) in said position.

14. Holding device (34, 36, 38, 40, 164, 180, 192, 200, 204) as defined in claims 12 and 13, **characterized in that** the first handling direction (116) and the second handling direction (118) are linearly independent of one another.

15. Holding device (34, 36, 38, 40, 164, 180, 192, 200, 204) as defined in any one of claims 3 to 13, **characterized in that** the holding device (34, 36, 38, 40, 164, 180, 192, 200, 204) defines a holding axis (48) predetermining the position and/or the location of the implant (42) when this is connected to the holding device (34, 36, 38, 40, 164, 180, 192, 200, 204).

16. Holding device (34, 36, 38, 40, 164, 180, 192, 200, 204) as defined in claim 15 and in any one of claims 13 or 14, **characterized in that** the holding axis (48) and the second handling direction (118) are parallel or essentially parallel to one another.

17. Holding device (34, 36, 38, 40, 164, 180, 192, 200, 204) as defined in any one of claims 3 to 16, **characterized in that** the first connecting device (44) comprises at least one holding element (62, 202) designed to connect the implant (42) to the holding device (34, 36, 38, 40, 164, 180, 192, 200, 204) in the first connecting position of the first connecting device (44).

18. Holding device (34, 36, 38, 40, 164, 180, 192, 200, 204) as defined in claims 3 and 17, **characterized in that** at least one holding element (62, 202) builds up a first restoring force for forming the first restoring device (73) during transfer of the first connecting device (44) from the first connecting position into the first release position, the first connecting device (46) being transferable back into the first connecting position with said restoring force.

19. Holding device (34, 36, 38, 40, 164, 180, 192, 200, 204) as defined in claim 17 or 18, **characterized in that** the at least one holding element (62, 202) limits an implant receptacle (58) for accommodating the implant (42).

20. Holding device (34, 36, 38, 40, 164, 180, 192, 204) as defined in claim 19, **characterized in that** the implant receptacle (58) has an undercut.

21. Holding device (180, 192, 200) as defined in any one of the preceding claims, **characterized in that** the indicating device (186) comprises at least one indicating element (188) adapted to be destroyed and/or plastically deformed during transfer of the first connecting device (44) from the first connecting position into the first release position.

22. Holding device (200) as defined in claim 21, **characterized in that** the at least one indicating element (188) is formed by a holding element (202).

23. Holding device (34, 36, 38, 40, 164, 180, 192, 200, 204) as defined in any one of the preceding claims, **characterized in that** it comprises a plate-like basic member (52).

24. Holding device (34, 36, 38, 40, 164, 180, 192, 200, 204) as defined in claims 19, 22 and 23, **characterized in that** the first connecting device comprises at least one contact element, which can abut on the implant in a tensioned manner in the first connecting position of the first connecting device and **in that** the at least one holding element (62, 202) and/or the at least one contact element (184) and/or the at least one indicating element (188) is or are designed in one piece with the basic member (52).

25. Holding device (34, 36, 38, 40, 164, 180, 192, 200, 204) as defined in any one of the preceding claims, **characterized in that** it comprises a data storage device (74) for storing implant data (76).

26. Holding device (34, 36, 38, 40, 164, 180, 192, 200, 204) as defined in any one of claims 2 to 11, 13 to 14, 16, 18 or in any one of claims 12, 15, 17, 19 to 25 and claim 2, **characterized in that** the second connecting device (46) comprises at least one connecting element (78, 122, 124) designed to connect the holding device (34, 36, 38, 40, 164, 180, 192, 200, 204) to the storage unit (2) in the second connecting position of the second connecting device (46).

27. Holding device (34, 36, 38, 40, 164, 180, 192, 200, 204) as defined in claim 26, **characterized in that** for forming a snap-in connection the at least one connecting element (78, 122, 124) comprises at least one snap-in element (80, 126, 128) or is designed as a snap-in element (80, 126, 128), wherein the snap-in element (80, 126, 128) is in engagement or is adapted to be brought into engagement interlockingly with the storage unit (2) in the second connecting position of the second connecting device (46).

28. Holding device (34, 36, 38, 40, 164, 180, 192, 200, 204) as defined in claim 26 or 27, **characterized in that** the at least one connecting element (78, 122, 124) is movable and/or deformable within a connecting plane (130).

29. Holding device (34, 36, 38, 40, 164, 180, 192, 200, 204) as defined in claim 6 and any one of claims 26 to 28, **characterized in that** the at least one connecting element (78, 122, 124) limits an undercut area (82) for accommodating a section (24, 30) of the storage unit (2) in the second connecting position of the second connecting device (46).

30. Holding device (40, 164, 180, 192, 200) as defined in any one of claims 26 to 29, **characterized in that** the second connecting device (46) comprises at least one essentially U-shaped material section (158) having two legs of the U (152, 156) extending parallel or essentially parallel to the holding axis (48), said legs being connected to one another via a base of the U (154).

31. Holding device (40, 164, 180, 192, 200, 204) as defined in claim 6 and any one of claims 7 to 30, **characterized in that** the second connecting device (46) has at least one actuating element (162, 166) for transferring the second connecting device (46) from the second connecting position into the second release position.

32. Holding device (34, 36, 38, 40, 164, 180, 192, 200, 204) as defined in any one of the preceding claims, **characterized in that** it comprises at least one securing device (88) preventing any implantation of the implant (42) when the implant (42) is connected to the holding device (34, 36, 38, 40, 164, 180, 192, 200, 204).

33. Holding device (34, 36, 38, 40, 164, 180, 192, 200, 204) as defined in any one of the preceding claims, **characterized in that** it has a guiding device (84) for positioning the holding device (34, 36, 38, 40, 164, 180, 192, 200, 204) relative to the storage unit (2).

34. Holding device (34, 36, 38, 40, 164, 180, 192, 200, 204) as defined in any one of the preceding claims with at least one implant (42).

35. Storage unit (2) for accommodating and/or securing in place at least one holding device (34, 36, 38, 40, 164, 180, 192, 200, 204) for an implant (42), which storage unit comprises a holding device (34, 36, 38, 40, 164, 180, 192, 200, 204) as defined in any one of the preceding claims.

36. Storage unit (2) as defined in claim 35, **characterized in that** it comprises at least one receptacle (16) for accommodating and/or securing at least one holding device (34, 36, 38, 40, 164, 180, 192, 200, 204) in place.

## Revendications

1. Dispositif de maintien (34, 36, 38, 40, 164, 180, 192, 200, 204) pour un implant (42), comprenant un premier système de liaison (44) pour assurer la liaison amovible du dispositif de maintien (34, 36, 38, 40, 164, 180, 192, 200, 204) avec l'implant (42), le premier système de liaison (44) pouvant être transféré d'une première position de liaison, dans laquelle un implant (42) peut être relié ou est relié au dispositif de maintien (34, 36, 38, 40, 164, 180, 192, 200, 204), à une première position de détachement, dans laquelle le dispositif de maintien (34, 36, 38, 40, 164, 180, 192, 200, 204) libère l'implant (42), **caractérisé en ce que** le dispositif de maintien comporte un dispositif indicateur (186), qui indique ou visualise un transfert au moins unique du premier système de liaison (44), de la première position de liaison à la première position de détachement.

2. Dispositif de maintien (34, 36, 38, 40, 164, 180, 192, 200, 204) selon la revendication 1, **caractérisé en ce qu'**il comporte un deuxième système de liaison (46) pour assurer la liaison amovible du dispositif de maintien (34, 36, 38, 40, 164, 180, 192, 200, 204) avec une unité de magasin de stockage (2).

3. Dispositif de maintien (34, 36, 38, 40, 164, 180, 192, 200, 204) selon la revendication 2, **caractérisé en ce que** le premier système de liaison (44) et le deuxième système de liaison (46) peuvent être actionnés indépendamment l'un de l'autre.

4. Dispositif de maintien (34, 36, 38, 40, 164, 180, 192, 200, 204) selon la revendication 3, **caractérisé en ce que** le premier système de liaison (44) est conçu de façon à ce que le transfert du premier système de liaison (44) de la première position de liaison à la première position de détachement, nécessite une première force de détachement.

5. Dispositif de maintien (34, 36, 38, 40, 164, 180, 192, 200, 204) selon la revendication 3 ou la revendication 4, **caractérisé en ce qu'**il comprend un premier système de rappel (73), qui transfère le premier système de liaison (44) de la première position de détachement à la première position de liaison.

6. Dispositif de maintien (34, 36, 38, 40, 164, 180, 192, 200, 204) selon l'une des revendications 2 à 5, **caractérisé en ce que** le deuxième système de liaison (46) peut être transféré d'une deuxième position de liaison, dans laquelle le dispositif de maintien (34, 36, 38, 40, 164, 180, 192, 200, 204) peut être relié ou est relié avec l'unité de magasin de stockage (2), à une deuxième position de détachement, dans laquelle le dispositif de maintien (34, 36, 38, 40, 164, 180, 192, 200, 204) peut être détaché de l'unité de magasin de stockage (2).

7. Dispositif de maintien (34, 36, 38, 40, 164, 180, 192, 200, 204) selon la revendication 6, **caractérisé en ce que** le deuxième système de liaison (46) est conçu de façon à ce que le transfert du deuxième système de liaison (46) de la deuxième position de liaison à la deuxième position de détachement, nécessite une deuxième force de détachement.

8. Dispositif de maintien (34, 36, 38, 40, 164, 180, 192, 200, 204) selon la revendication 6 ou la revendication 7, **caractérisé en ce qu'**il comprend un deuxième système de rappel (81), qui transfère le deuxième système de liaison (46) de la deuxième position de détachement à la deuxième position de liaison.

9. Dispositif de maintien (34, 36, 38, 40, 164, 180, 192, 200, 204) selon les revendications 4 et 7 ou selon les revendications 4, 7 et 8, **caractérisé en ce que** la première force de détachement et la deuxième force de détachement se distinguent l'une de l'autre quant à leur valeur et/ou à leur direction.

10. Dispositif de maintien (34, 36, 38, 40, 164, 180, 192, 200, 204) selon les revendications 4 et 7 ou selon les revendications 4, 7 ou 8 ou selon la revendication 9, **caractérisé en ce que** la première force de détachement et la deuxième force de détachement sont indépendantes linéairement l'une de l'autre.

11. Dispositif de maintien (34, 36, 38, 40, 164, 180, 192, 200, 204) selon les revendications 4 et 7 ou selon les revendications 4, 7 et 8 ou selon l'une des revendications 9 ou 10, **caractérisé en ce que** la première force de détachement est plus petite que la deuxième force de détachement.

12. Dispositif de maintien (34, 36, 38, 40, 164, 180, 192, 200, 204) selon l'une des revendications précédentes, **caractérisé en ce que** le premier système de liaison (44) est conçu de façon telle, que l'implant (42), lors d'un mouvement à partir d'une première position de maintien, dans laquelle l'implant (42) est relié au dispositif de maintien (34, 36, 38, 40, 164, 180, 192, 200, 204), vers une première position de libération, dans laquelle l'implant (42) est détaché du dispositif de maintien (34, 36, 38, 40, 164, 180, 192, 200, 204), puisse être manipulé dans une première direction de manipulation (116).

13. Dispositif de maintien (34, 36, 38, 40, 164, 180, 192, 200, 204) selon l'une des revendications 2 à 11 ou selon les revendications 2 et 12, **caractérisé en ce que** le deuxième système de liaison (46) est conçu de façon telle, que le dispositif de maintien (34, 36, 38, 40, 164, 180, 192, 200, 204), lors d'un mouvement à partir d'une deuxième position de maintien, dans laquelle le dispositif de maintien (34, 36, 38, 40, 164, 180, 192, 200, 204) est relié à l'unité de magasin de stockage (2), vers une deuxième position de libération, dans laquelle le dispositif de maintien (34, 36, 38, 40, 164, 180, 192, 200, 204) est détaché de l'unité de magasin de stockage (2), puisse être manipulé dans une deuxième direction de manipulation (118).

14. Dispositif de maintien (34, 36, 38, 40, 164, 180, 192, 200, 204) selon les revendications 12 et 13, **caractérisé en ce que** la première direction de manipulation (116) et la deuxième direction de manipulation (118) sont indépendantes linéairement l'une de l'autre.

15. Dispositif de maintien (34, 36, 38, 40, 164, 180, 192, 200, 204) selon l'une des revendications 3 à 13, **caractérisé en ce que** le dispositif de maintien (34, 36, 38, 40, 164, 180, 192, 200, 204) définit un axe de maintien (48), qui prédétermine la position et/ou la situation de l'implant (42) lorsque celui-ci est relié au dispositif de maintien (34, 36, 38, 40, 164, 180, 192, 200, 204).

16. Dispositif de maintien (34, 36, 38, 40, 164, 180, 192, 200, 204) selon la revendication 15 et l'une des revendications 13 ou 14, **caractérisé en ce que** l'axe de maintien (48) et la deuxième direction de manipulation (118) sont mutuellement parallèles ou sensiblement parallèles.

17. Dispositif de maintien (34, 36, 38, 40, 164, 180, 192, 200, 204) selon l'une des revendications 3 à 16, **caractérisé en ce que** le premier système de liaison (44) comprend au moins un élément de maintien (62, 202) qui est conçu pour, dans la première position de liaison du premier système de liaison (44), relier l'implant (42) au dispositif de maintien (34, 36, 38, 40, 164, 180, 192, 200, 204).

18. Dispositif de maintien (34, 36, 38, 40, 164, 180, 192, 200, 204) selon les revendications 3 et 17, **caractérisé en ce qu'**au moins un élément de maintien (62, 202), pour former le premier système de rappel (73), engendre, lors du transfert du premier système de liaison (44) de la première position de liaison à la première position de détachement, une première force de rappel avec laquelle le premier système de liaison (44) peut être transféré en retour dans la première position de liaison.

19. Dispositif de maintien (34, 36, 38, 40, 164, 180, 192, 200, 204) selon la revendication 17 ou la revendication 18, **caractérisé en ce que** ledit au moins un élément de maintien (62, 202) délimite un logement d'accueil d'implant (58) destiné à accueillir l'implant (42).

20. Dispositif de maintien (34, 36, 38, 40, 164, 180, 192, 204) selon la revendication 19, **caractérisé en ce que** le logement d'accueil d'implant (58) présente une contre-dépouille.

21. Dispositif de maintien (180, 192, 200) selon l'une des revendications précédentes, **caractérisé en ce que** le dispositif indicateur (186) comprend au moins un élément indicateur (188), qui, lors du transfert du premier système de liaison (44) de la première position de liaison à la première position de détachement, peut être détruit et/ou déformé de manière plastique.

22. Dispositif de maintien (200) selon la revendication 21, **caractérisé en ce que** ledit au moins un élément indicateur (188) est formé par un élément de maintien (202).

23. Dispositif de maintien (34, 36, 38, 40, 164, 180, 192, 200, 204) selon l'une des revendications précédentes, **caractérisé en ce qu'**il comporte un corps de base (52) en forme de plaque.

24. Dispositif de maintien (34, 36, 38, 40, 164, 180, 192, 200, 204) selon les revendications 19, 22 et 23, **caractérisé en ce que** le premier système de liaison comporte au moins un élément d'appui, qui, dans la première position de liaison du premier système de liaison, peut s'appliquer sous précontrainte contre l'implant, et **en ce que** ledit au moins un élément de maintien (62, 202) et/ou ledit au moins un élément d'appui (184) et/ou ledit au moins un élément indicateur (188) est ou sont réalisés d'un seul tenant avec le corps de base (52).

25. Dispositif de maintien (34, 36, 38, 40, 164, 180, 192, 200, 204) selon l'une des revendications précédentes, **caractérisé en ce qu'**il comporte un système de mémorisation de données (74) pour la mémorisation de données d'implant (76).

26. Dispositif de maintien (34, 36, 38, 40, 164, 180, 192, 200, 204) selon l'une des revendications 2-11, 13-14, 16, 18 ou selon l'une des revendications 12, 15, 17, 19-25 et la revendication 2, **caractérisé en ce que** le deuxième système de liaison (46) comprend au moins un élément de liaison (78, 122, 124), qui est conçu pour, dans la deuxième position de liaison du deuxième système de liaison (46), relier le dispositif de maintien (34, 36, 38, 40, 164, 180, 192, 200, 204) à l'unité de magasin de stockage (2).

27. Dispositif de maintien (34, 36, 38, 40, 164, 180, 192, 200, 204) selon la revendication 26, **caractérisé en ce que** ledit au moins un élément de liaison (78, 122, 124), en vue de former une liaison par encliquetage, comporte au moins un élément d'encliquetage (80, 126, 128) ou est réalisé en tant qu'élément d'encliquetage (80, 126, 128), l'élément d'encliquetage (80, 126, 128) étant ou pouvant être amené en prise par encliquetage avec l'unité de magasin de stockage (2), dans la deuxième position de liaison du deuxième système de liaison (46).

28. Dispositif de maintien (34, 36, 38, 40, 164, 180, 192, 200, 204) selon la revendication 26 ou la revendication 27, **caractérisé en ce que** ledit au moins un élément de liaison (78, 122, 124) est mobile et/ou déformable à l'intérieur d'un plan de liaison (130).

29. Dispositif de maintien (34, 36, 38, 40, 164, 180, 192, 200, 204) selon la revendication 6 et l'une des revendications 26 à 28, **caractérisé en ce que** ledit au moins un élément de liaison (78, 122, 124), dans la deuxième position de liaison du deuxième système de liaison (46), délimite une zone de contre-dépouille (82) en vue d'accueillir un tronçon (24, 30) de l'unité de magasin de stockage (2).

30. Dispositif de maintien (40, 164, 180, 192, 200) selon l'une des revendications 26 à 29, **caractérisé en ce que** le deuxième système de liaison (46) comporte au moins un tronçon de matériau (158) sensiblement en forme U présentant deux ailes de U (152, 156), qui s'étendent parallèlement ou sensiblement de manière parallèle à l'axe de maintien (48), et qui sont reliées mutuellement par l'intermédiaire d'une base de U (154).

31. Dispositif de maintien (40, 164, 180, 192, 200, 204) selon la revendication 6 et l'une des revendications 7 à 30, **caractérisé en ce que** le deuxième système de liaison (46) comporte au moins un élément d'actionnement (162, 166) pour transférer le deuxième système de liaison (46) de la deuxième position de liaison à la deuxième position de détachement.

32. Dispositif de maintien (34, 36, 38, 40, 164, 180, 192, 200, 204) selon l'une des revendications précédentes, **caractérisé en ce qu'**il comprend au moins un système de sécurité (88), qui empêche d'implanter l'implant (42) lorsque l'implant (42) est relié au dispositif de maintien (34, 36, 38, 40, 164, 180, 192, 200, 204).

33. Dispositif de maintien (34, 36, 38, 40, 164, 180, 192, 200, 204) selon l'une des revendications précédentes, **caractérisé en ce qu'**il comporte un système de guidage (84) à l'aide duquel le dispositif de maintien (34, 36, 38, 40, 164, 180, 192, 200, 204) peut être positionné par rapport à l'unité de magasin de stockage (2).

34. Dispositif de maintien (34, 36, 38, 40, 164, 180, 192, 200, 204) selon l'une des revendications précédentes, comprenant au moins un implant (42).

35. Unité de magasin de stockage (2) destinée à accueillir et/ou fixer au moins un dispositif de maintien (34, 36, 38, 40, 164, 180, 192, 200, 204) pour un implant (42), ladite unité de magasin de stockage comprenant un dispositif de maintien (34, 36, 38, 40, 164, 180, 192, 200, 204) selon l'une des revendications précédentes.

36. Unité de magasin de stockage (2) selon la revendication 35, **caractérisée en ce qu'**elle comporte au moins un logement d'accueil (16) destiné à accueillir et/ou fixer au moins un dispositif de maintien (34, 36, 38, 40, 164, 180, 192, 200, 204).
